# EUROPÄISCHE PATENTSCHRIFT

(11) **EP 0 973 756 B1**
(45) Veröffentlichungstag und Bekanntmachung des Hinweises auf die Patenterteilung: **26.03.2003**
(21) Anmeldenummer: 98917025.3
(22) Anmeldetag: 20.03.1998
(51) Int. Cl.: C07D 273/00, A61K 31/395

(54) **THIODEPSIPEPTIDE ZUR BEKÄMPFUNG VON ENDOPARASITEN UND EIN VERFAHREN ZU IHRER HERSTELLUNG**
THIODEPSIPEPTIDES FOR COMBATING ENDOPARASITES AND A METHOD FOR PRODUCING THE SAME
THIODEPSIPEPTIDES POUR LUTTER CONTRE DES ENDOPARASITES ET PROCEDE SIMPLE PERMETTANT DE LES PREPARER

(30) Priorität: 02.04.1997 DE 19713626
(43) Veröffentlichungstag der Anmeldung: 26.01.2000
(73) Patentinhaber: BAYER AG, 51368 Leverkusen (DE)
(72) Erfinder: JESCHKE, Peter, D-51373 Leverkusen (DE); HARDER, Achim, D-51109 Köln (DE); VON SAMSON-HIMMELSTJERNA, Georg, D-42657 Solingen (DE); MENCKE, Norbert, D-51381 Leverkusen (DE); BONSE, Gerhard, D-51061 Köln (DE); IINUMA, Katsuharu, Odawara-shi, Kanagawa-ken 250 (JP); SAKANAKA, Osamu, Odawara-shi, Kanagawa-ken 250 (JP)
(74) Vertreter: Bader, Axel Jochen
(86) Internationale Anmeldenummer: EP9801628
(87) Internationale Veröffentlichungsnummer: WO98043965

(56) Entgegenhaltungen:
- EP-A- 0 382 173
- EP-A- 0 503 538
- EP-A- 0 626 375
- EP-A- 0 626 376
- EP-A- 0 634 408
- EP-A- 0 685 469
- EP-A- 0 718 293
- WO-A-96/11945

## Beschreibung

Die vorliegende Erfindung betrifft cyclische Thiodepsipeptide, insbesondere 18- bis 24-gliedrige Cyclothiodepsipeptide, ein einfaches Verfahren zu ihrer Herstellung und ihre Verwendung zur Bekämpfung von Endoparasiten.

Zu den bevorzugten Strukturvariationen von biologisch aktiven Peptiden zählt der Austausch von Amid-Sauerstoff gegen Schwefel, da die Thioamidgruppe (ψ[CSNH]) deutliche Isosterie zur Amidgruppe (CONH) aufweist (vgl. z. B. D. B. Sherman et al., J. Amer. Soc. 112, 1990, S. 433; M. Kajtar et al., Tetrahedron 42, 1986, S. 3931; D. J. S. Guthrie et al., Int. J. Peptide Protein Res. 28, 1986, S. 208).

Allerdings ist die Vorhersage der zu erwartenden biologischen Potenz des Thiopeptids, trotz hoher physikalischer Ähnlichkeit zum Ausgangspeptid, schwierig. (vgl. L. Lankiewics et al., Biochem. Biophys. Res. Commun. 184, (1), 1992, S. 359).

Methoden zur Herstellung verschiedener Endothiopeptide sind aus der Literatur bekannt. Beispielhaft seien hier die Thionierungsstrategien von unterschiedlichen Hormonen wie dem neurohypophysären Hormon Oxytocin, dem Thyreotropin Releasing-Hormon (TRH), dem Wachstumshormon Releasing-Peptide, den Neuropeptiden Leucin-Enkephalin und Demorphin genannt. (vgl. [1-Deamino,9-thioglycin] oxytocin: W. C. Jones et al., J. Amer. Chem. Soc. 95, 1973, S. 5677; Thio-TRH: Zs. Majer et al., Biochem. Biophys. Res. Commun. 150, 1988, S. 1017; thionierte Leucin-Enkephaline: K. Clausen et al., J. Chem. Soc., Perkin Trans. I 1984, S. 785 sowie Biochem. Biophys. Res. Commun. 120, 1984, S. 305; Thio-Demorphin: S. Salvadori et al., Farmaco Ed. Sc., 39, 1984, S. 216; Thio-TRH: M. Kruszynski et al., Experentia 41, 1985, S. 1576).

Von den Autoren Ried *et al*., Mock *et al*., Campbell *et al.* oder Bartlett *et al.* wurden weitere Darstellungsmöglichkeiten von N-geschützten Endothiodipeptidestern und deren Salze erarbeitet, Brown *et al.* gelang der Zugang zu N-geschützten Endothiotripeptidestem (vgl. W. Ried et al., Angew. Chem. 72, 1960, S. 268; W. L. Mock et al., Biochem. Biophys. Res. Commun. 102, 1981, S. 389; 1973, S. 5677; Campbell et al., J. Amer. Chem. Soc. 104, 1982, S. 5221; P. A. Bartlett et al., 21, 1982, S. 1608; D. W. Brown et al., Tetrahedron 39, 1983, 1075).

Verglichen mit den bereits genannten Verfahren zur Herstellung verschiedener Endothiopeptide ist über die Darstellung von Thiodepsipeptiden bestehend aus Aminosäuren, Hydroxythiocarbonsäuren und gegebenenfalls Hydroxycarbonsäuren, nur wenig bekannt.

Bekannt ist die Herstellung und Verwendung einiger Thiolester von Aminosäuren als Insektizide und Akarizide (vgl. DE-OS 2812169, C. A. Henrick et al., Pestic. Sci. 11, (2), 1980, S. 224; US-Pat. 4 243 819, US-Pat. 4 231 953).

Weiterhin ist ein Thiodepsipeptid PM-93135, welches als Fermentationsprodukt aus einem Micromonospora-Stamm (Actinomyceten) isoliert wurde und antibakterielle Aktivität aufweist, bekannt (vgl. WO 95/277730).

Cyclothiodepsipeptide, bestehend aus Aminosäuren, Hydroxythiocarbonsäuren und gegebenenfalls Hydroxycarbonsäuren als Ringbausteine sind bisher nicht bekannt.

Cyclodepsipeptide mit 18 bis 24 Ringatomen als Mittel zur Bekämpfung von Endoparasiten sind bekannt (vgl. z. B. Y. Kodama et al., Sci. Reports of Meiji Seika Kaisha 31, 1992, S. 1-8; EP-OS 382 173; EP-OS 503 538; WO 93/ 19 053; WO 94/19334; WO 95/07272; EP 626 375; EP 626 376; EP 664 297; EP 634 408; EP 718 298).

WO 96/11945 beschreibt ein Verfahren zur aromatischen Sulfonylierung, Sulfenylierung, Thiocyanierung und Phosphorylierung von cyclischen Depsipeptiden mit 6 bis 24 Ringatomen; cyclische Thiodepsipeptide werden darin nicht offenbart.

Gegenstand der vorliegenden Erfindung sind neue cyclische Thiodepsipeptide und ein Verfahren zur Darstellung der cyclischen Thiodepsipeptide, bestehend aus Aminosäuren, Hydroxythiocarbonsäuren und gegebenenfalls Hydroxycarbonsäuren als Ringbausteine und 18 bis 24 Ringatomen.

Ein weiterer Gegenstand ist auch die Verwendung von cyclischen Thiodepsipeptiden, bestehend aus Aminosäuren, Hydroxythiocarbonsäuren und gegebenenfalls Hydroxycarbonsäuren als Ringbausteine und 18 - 24 Ringatomen, als Mittel zur Bekämpfung von Endoparasiten.

Die vorliegende Erfindung betrifft insbesondere:
1. Verbindungen der allgemeinen Formel (I) und deren Salze 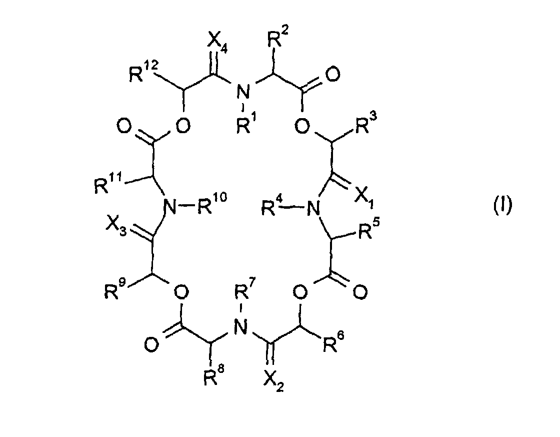 in welcher
   - R¹, R⁴, R⁷ und R¹⁰: für C₁₋₄-Alkyl, stehen,
   - R², R⁵, R⁸ und R¹¹: für C₁₋₄-Alkyl, stehen,
   - R³ und R⁹: für C₁₋₄-Alkyl, stehen,
   - R⁶ und R¹²: unabhängig voneinander für C₁₋₄-Alkyl, Benzothiazol-2-yl-methyl, C₁-C₄- Alkoxycarbonylmethyl, Diphenylmethoxycarbonylmethyl und für gegebenenfalls substituiertes Benzyl, wobei als Substituenten genannt seien Wasserstoff, Halogen, Cyan, Carbamoyl, C₁₋₄-Alkyl, eine Schutzgruppe tragendes Hydroxy oder Hydroxy, C₁₋₈-Alkoxy, Triphenylmethoxy, C₁₋₄-Alkoxy-C₁₋₄-alkoxy, C₂₋₄-Alkenyloxy,
   Hetaryl-C₁₋₄-alkoxy, wobei die Heterocyclen wiederum mit Substituenten aus der Reihe C₁₋₄-Alkyl, C₃₋₆-Cycloalkyl und Halogen tragen können, ausgewählt aus: Fur-2-yl-methoxy, Fur-3-yl-methoxy, Tetrahydrofur-2-ylmethoxy, N-Boc-pyrrolidin-2-yl-methoxy, Pyrrolidin-2-yl-methoxy, 5-sec-Butyl-1,2,4-oxadiazol-3-yl-pyridyl-methoxy, 5-Cyclopropyl-1,2,4-oxadiazol-3-yl-methoxy, Imidazol-5-yl-methoxy, Thiazolyl-methoxy, Tetrazol-5-ylmethoxy;
   Nitro, eine Schutzgruppe tragendes Amino oder Amino, eine Schutzgruppe tragendes Amino-C₁₋₆-alkoxy oder Amino-C₁₋₆-alkoxy, N-Mono-C₁₋₆alkylamino-C₁₋₆-alkoxy, N,N-Di-C₁₋₆-alkylamino-C₁₋₆-alkoxy, N,N-Di-[(C₁₋₆alkoxy-C₁₋₆-alkyl)]amino-C₁₋₆-alkoxy, C₁₋₄-Alkylamino, C₁₋₄-Dialkylamino, C₃₋₇-Cycloalkylamino- (wobei als ringbildende Atome ein oder mehr Stickstoffatome enthalten und darüber hinaus Sauerstoff- oder Schwefelatome enthalten sein können) C₁₋₆-alkoxy, 2-N-Morpholino-ethoxy, 2-N-Piperidino-ethoxy, Pyrrolidinomethoxy, C₃₋₇-Cyclo-alkylamino- (wobei als ringbildende Atome ein oder mehr Stickstoffatome enthalten und darüber hinaus Sauerstoff- oder Schwefelatome enthalten sein können), C₃₋₇-Cycloalkylamino- (wobei als ringbildende Atome ein oder mehr Stickstoffatome enthalten und darüber hinaus Sauerstoff- oder Schwefelatome enthalten sein können) sulfonyl, N-(N¹-Methyl-piperazino)-sulfonyl, N-(N¹-Methoxycarbonyl-piperazino)-sulfonyl, N¹-Imidazolyl-sulfonyl, N,N-Di-[(C₁₋₆-alkoxy-C₁₋₆-alkyl)]amino-sulfonyl, C₃₋₇-Cyclo-alkylamino- (wobei als ringbildende Atome ein oder mehr Stickstoffatome enthalten und darüber hinaus Sauerstoff- oder Schwefelatome enthalten sein können)-C₁₋₆-alkylamino-sulfonyl, C₁₋₄-Dialkylaminosulfonyl, Sulfamidyl,
   und
   (i)
      X¹ Schwefel bedeutet,
      X², X³ und X⁴ unabhängig voneinander Sauerstoff oder Schwefel bedeuten,
      oder
   (ii)
      X² Schwefel bedeutet,
      X¹, X³ und X⁴ unabhängig voneinander Sauerstoff oder Schwefel bedeuten,
      oder
   (iii)
      X³ Schwefel bedeutet,
      X¹, X² und X⁴ unabhängig voneinander Sauerstoff oder Schwefel bedeuten,
      oder
   (iv)
      X⁴ Schwefel bedeutet,
      X¹, X² und X³ unabhängig voneinander Sauerstoff oder Schwefel bedeuten,
   sowie deren optische Isomere und Racemate.
2. Die neuen cyclischen Thiodepsipeptide der allgemeinen Formel ( I ) und deren Salze, 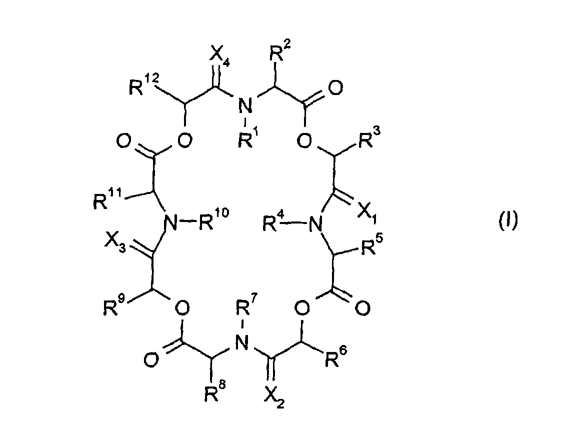 in welcher
   R¹ bis R¹² und X¹ bis X⁴ die unter Punkt 1 angegebenen Bedeutungen besitzen,
   werden hergestellt, indem man
   die Depsipeptide der allgemeinen Formel (II) und deren Salze, 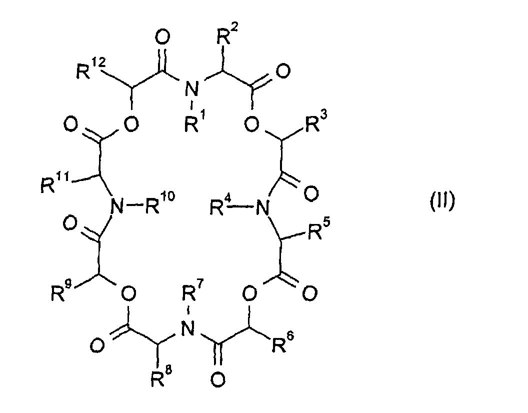 in welcher
   R¹ bis R¹² und X¹ bis X⁴ die unter Punkt 1 angegebenen Bedeutungen besitzen,
   in Gegenwart eines geeigneten Schweflungsagens und in Gegenwart eines geeigneten Verdünnungsmittels thioniert.

Die Verbindungen der allgemeinen Formel (I) können in Abhängigkeit von der Art der Substituenten als geometrische und/oder optische Isomerengemische unterschiedlicher Zusammensetzung auftreten. Die Erfindung betrifft sowohl die reinen Isomeren als auch die Isomerengemische.

Die erfindungsgemäßen Thiodepsipeptide und deren Salze sind durch die allgemeine Formel (I) allgemein definiert.

Die erfindungsgemäßen Thiodepsipeptide und deren Säureadditionssalze und Metallsalzkomplexe besitzen gute endoparasitizide, insbesondere anthelmintische Wirkung und können bevorzugt im Bereich der Veterinärmedizin eingesetzt werden.

Gegebenenfalls substituiertes Alkyl allein oder als Bestandteil eines Restes in den allgemeinen Formeln bedeutet geradkettiges oder verzweigtes Alkyl mit vorzugsweise 1 bis 6, insbesondere 1 bis 4 Kohlenstoffatomen. Beispielhaft seien gegebenenfalls substituiertes Methyl, Ethyl, n-Propyl, Isopropyl, n-Butyl, Isobutyl, sec.-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methyl-pentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1,2,2-Trimethylpropyl, 1-Ethylbutyl und Ethylbutyl genannt.

Vorzugsweise seien Methyl, Ethyl, n-Propyl, Isopropyl, n-Butyl, Isobutyl, sec-Butyl und tert-Butyl genannt.

Gegebenenfalls substituiertes Alkenyl allein oder als Bestandteil eines Restes in den allgemeinen Formeln bedeutet geradkettiges oder verzweigtes Alkenyl mit vorzugsweise 1 bis 6, insbesondere 1 bis 4 Kohlenstoffatomen. Beispielhaft seien gegebenenfalls substituiertes Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-bute-nyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-pro-penyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Me-thyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl genannt.

Vorzugsweise seien gegebenenfalls substituiertes Ethenyl, 2-Propenyl, 2-Butenyl oder 1-Methyl-2-propenyl genannt.

Gegebenenfalls substituiertes Alkinyl allein oder als Bestandteil eines Restes in den allgemeinen Formeln bedeutet geradkettiges oder verzweigtes Alkinyl mit vorzugsweise 1 bis 6, insbesondere 1 bis 4 Kohlenstoffatomen. Beispielhaft seien gegebenenfalls substituiertes Ethinyl, 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 11-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butinyl, 1,2-Dimethyl-3-butinyl, 2,2-Dimethyl-3-butinyl, 1-Ethyl-3-butinyl, 2-Ethyl-3-butinyl und 1-Ethyl-1-methyl-2-propinyl genannt.

Vorzugsweise seien gegebenenfalls substituiertes Ethinyl, 2-Propinyl oder 2-Butinyl genannt.

Gegebenenfalls substituiertes Cycloalkyl allein oder als Bestandteil eines Restes in den allgemeinen Formeln bedeutet mono-, bi- und tricyclisches Cycloalkyl, vorzugsweise mit 3 bis 10, insbesondere 3, 5 oder 7 Kohlenstoffatomen. Beispielhaft seien gegebenenfalls substituiertes Cyclopropyl, Cyclobutyl, Cyclopentyl, Cyclohexyl, Cycloheptyl, Cyclooktyl, Bicyclo[2.2.1]heptyl, Bicyclo[2.2.2]oktyl und Adamantyl genannt.

Halogenalkyl allein oder als Bestandteil eines Restes in den allgemeinen Formeln enthält 1 bis 4, insbesondere 1 oder 2 Kohlenstoffatome mit vorzugsweise 1 bis 9, insbesondere 1 bis 5 gleiche oder verschiedene Halogenatome vorzugsweise Fluor, Chlor oder Brom, insbesondere Fluor und Chlor. Beispielhaft seien Trifluormethyl, Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl und Pentafluor-tert-butyl genannt.

Gegebenenfalls substituiertes Alkoxy allein oder als Bestandteil eines Restes in den allgemeinen Formeln bedeutet geradkettiges oder verzweigtes Alkoxy mit vorzugsweise 1 bis 6, insbesondere 1 bis 4 Kohlenstoffatomen. Beispielhaft seien gegebenenfalls substituiertes Methoxy, Ethoxy, n-Propoxy, Isopropoxy, n-Butoxy, Isobutoxy, sec-Butoxy und tert-Butoxy genannt.

Gegebenenfalls substituiertes Alkoxyalkoxy allein oder als Bestandteil eines Restes in den allgemeinen Formeln bedeutet geradkettiges oder verzweigtes Alkoxyalkoxy mit vorzugsweise 1 bis 6, insbesondere 1 bis 4 Kohlenstoffatomen. Beispielhaft seien gegebenenfalls substituiertes Methoxymethoxy, Methoxyethoxy, Methoxy-npropoxy und Ethoxyisopropoxy genannt.

Gegebenenfalls substituiertes Alkoxyalkoxyalkoxy allein oder als Bestandteil eines Restes in den allgemeinen Formeln bedeutet geradkettiges oder verzweigtes Alkoxyalkoxyalkoxy mit vorzugsweise 1 bis 6, insbesondere 1 bis 4 Kohlenstoffatomen. Beispielhaft seien gegebenenfalls substituiertes Methoxymethoxyethoxy, Methoxyethoxyethoxy und Methoxyethoxy-n-propoxy genannt.

Gegebenenfalls substituiertes Halogenalkoxy allein oder als Bestandteil eines Restes in den allgemeinen Formeln bedeutet geradkettiges oder verzweigtes Halogenalkoxy mit vorzugsweise 1 bis 6, insbesondere 1 bis 4 Kohlenstoffatomen. Beispielhaft seien gegebenenfalls substituiertes Difluormethoxy, Trifluormethoxy, Trichlormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy und 2-Chlor-1,1,2-trifluorethoxy genannt.

Gegebenenfalls substituiertes Alkylthio allein oder als Bestandteil eines Restes in den allgemeinen Formeln bedeutet geradkettiges oder verzweigtes Alkylthio mit vorzugsweise 1 bis 6, insbesondere 1 bis 4 Kohlenstoffatomen. Beispielhaft seien gegebenenfalls substituiertes Methylthio, Ethylthio, n-Propylthio, Isopropylthio, n-Butylthio, Isobutylthio, sec-Butylthio und tert-Butylthio genannt.

Gegebenenfalls substituiertes Halogenalkylthio allein oder als Bestandteil eines Restes in den allgemeinen Formeln bedeutet geradkettiges oder verzweigtes Halogenalkylthio mit vorzugsweise 1 bis 6, insbesondere 1 bis 4 Kohlenstoffatomen. Beispielhaft seien gegebenenfalls substituiertes Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio und 2-Chlor-1,1,2-trifluorethylthio genannt.

Gegebenenfalls substituiertes Alkylcarbonyl allein oder als Bestandteil eines Restes in den allgemeinen Formeln bedeutet geradkettiges oder verzweigtes Alkylcarbonyl mit vorzugsweise 1 bis 6, insbesondere 1 bis 4 Kohlenstoffatomen. Beispielhaft seien gegebenenfalls substituiertes Methylcarbonyl, Ethylcarbonyl, n-Propylcarbonyl, Isopropylcarbonyl, sec-Butylcarbonyl und tert-Butylcarbonyl genannt.

Gegebenenfalls substituiertes Cycloalkylcarbonyl allein oder als Bestandteil eines Restes in den allgemeinen Formeln bedeutet mono-, bi- und tricyclisches Cycloalkylcarbonyl, vorzugsweise mit 3 bis 10, insbesondere 3, 5 oder 7 Kohlenstoffatomen. Beispielhaft seien gegebenenfalls substituiertes Cyclopropylcarbonyl, Cyclobutylcarbonyl, Cyclopentylcarbonyl, Cyclohexylcarbonyl, Cycloheptylcarbonyl, Cyclooktylcarbonyl, Bicyclo[2.2.1]heptylcarbonyl, Bicyclo[2.2.2]oktylcarbonyl und Adamantylcarbonyl genannt.

Gegebenenfalls substituiertes Alkoxycarbonyl allein oder als Bestandteil eines Restes in den allgemeinen Formeln bedeutet geradkettiges oder verzweigtes Alkoxy mit vorzugsweise 1 bis 6, insbesondere 1 bis 4 Kohlenstoffatomen. Beispielhaft seien gegebenenfalls substituiertes Methoxycarbonyl, Ethoxycarbonyl, n-Propoxycarbonyl, Isopropoxycarbonyl, n-Butoxycarbonyl, Isobutoxycarbonyl, sec-Butoxycarbonyl und tert-Butoxycarbonyl genannt.

Aryl ist beispielsweise ein ein-, zwei- oder mehrkerniger aromatischer Rest wie Phenyl, Naphthyl, Tetrahydronaphthyl, Indanyl, Fluorenyl und ähnliches, vorzugsweise aber Phenyl oder Naphthyl.

Gegebenenfalls substituiertes Aryl in den allgemeinen Formeln bedeutet vorzugsweise gegebenenfalls substituiertes Phenyl oder Naphthyl, insbesondere Phenyl.

Gegebenenfalls substituiertes Arylalkyl in den allgemeinen Formeln bedeutet vorzugsweise gegebenenfalls im Arylteil und/oder Alkyl substituiertes Arylalkyl mit vorzugsweise 6 oder 10, insbesondere 8 Kohlenstoffatomen im Arylteil (vorzugsweise Phenyl oder Naphthyl, insbesondere Phenyl) und vorzugsweise 1 bis 4, insbesondere 1 oder 2 Kohlenstoffatomen im Alkylteil, wobei der Alkylteil geradkettig oder verzweigt sein kann. Beispielhaft und vorzugsweise seien gegebenenfalls substituiertes Benzyl und 1-Phenylethyl genannt.

Die gegebenenfalls substituierten Reste der allgemeinen Formeln können einen oder mehrere, vorzugsweise 1 bis 3, insbesondere 1 bis 2 gleiche oder verschiedene Substituenten tragen. Als Substituenten seien beispielhaft und vorzugsweise aufgeführt:
Alkyl mit vorzugsweise 1 bis 4, insbesondere 1 bis 2 Kohlenstoffatomen, wie Methyl, Ethyl, n-Propyl, Isopropyl, n-Butyl, Isobutyl, sec-Butyl und tert-Butyl; Alkoxy mit vorzugsweise 1 bis 4, insbesondere 1 bis 2 Kohlenstoffatomen, wie Methoxy, Ethoxy, n-Propoxy, Isopropoxy, n-Butoxy, Isobutoxy, sec-Butoxy und tert-Butoxy; Alkylthio wie Methylthio, Ethylthio, n-Propylthio, Isopropylthio, n-Butylthio, Isobutylthio, sec-Butylthio; Halogenalkyl mit vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor oder Chlor stehen, wie Difluormethyl, Trifluormethyl, Trichlormethyl; Hydroxy; Halogen, vorzugsweise Fluor, Chlor, Brom und Iod, insbesondere Fluor und Chlor; Cyan; Nitro; Amino; Monoalkyl- und Dialkylamino mit vorzugsweise 1 bis 4, insbesondere 1 oder 2 Kohlenstoffatomen je Alkylgruppe, wie Methylamino, Methylethylamino, Dimethylamino, n-Propylamino, Isopropylamino, Methyl-n-butylamino; Alkylcarbonylreste wie Methylcarbonyl; Alkoxycarbonyl mit vorzugsweise 2 bis 4, insbesondere 2 bis 3 Kohlen-stoffatomen wie Methoxycarbonyl und Ethoxycarbonyl; Alkylsulfinyl mit 1 bis 4, insbesondere 1 bis 2 Kohlenstoffatomen; Halogenalkylsulfinyl mit 1 bis 4, insbesondere 1 bis 2 Kohlenstoffatomen und 1 bis 5 Halogenatomen wie Trifluormethylsulfinyl; Halogenalkylsulfonyl mit 1 bis 4, insbesondere 1 bis 2 Kohlenstoffatomen und 1 bis 5 Halogenatomen wie Trifluormethylsulfonyl, Perfluor-n-butylsulfonyl, Perfluorisobutylsulfonyl; Arylsulfonyl mit vorzugsweise 6 oder 10 Arylkohlenstoffatomen wie Phenylsulfonyl; Acyl, Aryl, Aryloxy, die ihrerseits einen der obengenannten Substituenten tragen können sowie den Formiminorest (-HC=N-O-Alkyl).

Die Anzahl dieser Substituenten ist nicht begrenzt, sie liegt vorzugsweise bei ein bis vier gleichen oder unterschiedlichen Substituenten. Auch das Vorhandensein von zwei gleichen oder unterschiedlichen Substituenten am gleichen Atom oder an Atomen cyclischer Aminogruppen ist denkbar.

Gegebenenfalls substituierte Mono- oder Dialkylaminogruppen allein oder als Bestandteil eines Restes in den allgemeinen Formeln bedeutet geradkettiges oder verzweigtes Alkyl mit vorzugsweise 1 bis 6, insbesondere 1 bis 4 Kohlenstoffatomen. Beispielhaft für substituierte Mono- oder Dialkylaminogruppen seien Methylamino, Ethylamino, Dimethylamino, Diethylamino, Di-n-propylamino, Diisopropylamino oder Dibutylamino genannt.

Gegebenenfalls substituierte Mono- oder Dialkoxyalkylaminogruppen allein oder als Bestandteil eines Restes in den allgemeinen Formeln bedeutet geradkettiges oder verzweigtes Alkoxyalkyl mit vorzugsweise 1 bis 6, insbesondere 1 bis 4 Kohlenstoffatomen. Beispielhaft fiir substituierte Mono- oder Dialkoxyalkyl-aminogruppen seien Methoxymethylamino, Methoxyethylamino, Di-(methoxymethyl)-amino oder Di-(methoxyethyl)-amino genannt.

Als geeignete cyclische Aminogruppen kommen heteroaromatische oder aliphatische Ringsysteme mit einem oder mehreren Stickstoffatomen als Heteroatom in Frage, bei denen die Heterocyclen gesättigt oder ungesättigt, ein Ringsystem oder mehrere kondensierte Ringsysteme sein können, und gegebenenfalls weitere Heteroatome wie beispielsweise ein oder zwei Stickstoff, Sauerstoff und Schwefel usw. enthalten. Außerdem können cyclische Aminogruppen auch ein Spiroring oder verbrücktes Ringsystem bedeuten. Die Anzahl der Atome, die cyclische Aminogruppen bilden, ist nicht begrenzt, beispielsweise bestehen sie im Falle eines Einringsystems aus 3 bis 8 Atomen und im Falle eines Dreiringsystems aus 7 bis 11 Atomen.

Beispielhaft für cyclische Aminogruppen mit gesättigten und ungesättigten monocyclischen Gruppen mit einem Stickstoffatom als Heteroatom seien 1-Azetidinyl, Pyrrolidino, 2-Pyrrolin-1-yl, 1-Pyrrolyl, Piperidino, 1,4-Dihydropyrazin-1-yl, 1,2,5,6-Tetrahydropyrazin-1-yl, 1,4-Dihydropyridin-1-yl, 1,2,5,6-Tetrahydropyridin-1-yl, Homopiperidinyl genannt; beispielhaft für cyclische Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-pyridazin-1-yl, 1,2-Dihydropyrimidin-1-yl, Perhydropyrimidin-1-yl, 1,4-Diazacyclo-heptan-1-yl genannt; beispielhaft für cyclische Aminogruppen mit gesättigten und ungesättigten monocyclischen Gruppen mit einem oder zwei Sauerstoffatomen und einem bis 3 Stickstoffatomen als Heteroatome, wie beispielsweise Oxazolidin-3-yl, 2,3-Dihydroisoxazol-2-yl, Isoxazol-2-yl, 1,2,3-Oxadiazin-2-yl, Morpholino, beispielhaft für cyclische Aminogruppen mit gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino, oder Dioxothiomorpholino genannt; beispielhaft für cyclische Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclische Aminogruppen mit spirocyclischen Gruppen sei das 2-Azaspiro[4,5]decan-2-yl genannt; beispielhaft für cyclische Aminogruppen mit verbrückten heterocyclischen Gruppen sei das 2-Azabicyclo[2,2,1]heptan-7-yl genannt.

Als geeignete Aminoschutzgruppen kommen Acylgruppen, mit vorzugsweise 1 bis 6, insbesondere 1 bis 4 Kohlenstoffatomen, wie beispielsweise Formyl, Acetyl, Propionyl, Pivaloyl, Hexanoyl oder mono- (oder di- bzw. tri-) Halogen-haltige Acylgruppen, wie beispielsweise Chloracetyl, Bromacetyl, Dichloracetyl und Trifluoracetyl, Alkoxycarbonylgruppe mit vorzugsweise 1 bis 14, insbesondere 1 bis 4 Kohlenstoffatomen, wie beispielsweise Methoxycarbonyl, Ethoxycarbonyl, Propoxycarbonyl, tert-Butoxycarbonyl (Boc), tert-Amyloxycarbonyl (Aoc), Hexyloxycarbonyl, Methylsulfonylethoxycarbonyl, Adamantyloxycarbonyl (Adoc) und 1-[1-Adamantyl]-1-methylethoxycarbonyl (Adpoc), Carbamoylgruppen, Aroylgruppen, wie beispielsweise Phenylacetyl und Phenylpropionyl, Aryloxycarbonylgruppen, wie beispielsweise Phenoxycarbonyl und Naphthyloxycarbonyl, Aryloxyalkanoylgruppen, wie beispielsweise Phenoxyacetyl, und Phenoxypropionyl, Arylglyoxyloylgruppen, wie beispielsweise Phenylglyoxyloyl und Naphthylglyoxyloyl, Alkoxycarbonylgruppen mit gebräuchlichen Substituenten, wie beispielsweise Benzyloxycarbonyl (Cbo- bzw. Cbz, Z), 4-Methoxybenzyloxycarbonyl, 3,5-Dimethoxy-benzyloxycarbonyl, 4-Phenylazo-benzyloxycarbonyl, Phenethyloxycarbonyl, Nitro-benzyloxycarbonyl, Chlor-benzyloxycarbonyl, α,α-Dimethyl-3,5-dimethoxy-benzyloxycarbonyl, 2-Nitro-4,5-dimethoxybenzyloxycarbonyl (Nvoc), Fluorenyl-9-methoxycarbonyl (Fmoc), substituierte oder unsubstituierte Alkylidengruppen, wie beispielsweise Benzyliden, Hydroxybenzyliden, mono- (oder di- bzw. tri-) Phenylalkyl-haltige Alkylgruppen, wie beispielsweise Benzyl, Phenethyl, Benzhydryl oder Triphenylmethyl (Trityl), und ähnliche, in Frage.

Als geeignete Hydroxyschutzgruppen kommen gegebenenfalls substituierte Alkylgruppen mit vorzugsweise 1 bis 6, insbesondere 1 bis 4 Kohlenstoffatomen, wie beispielsweise tert-Butyl, Methylthiomethyl und Trimethylsilyl, Phenylalkyl-haltige Alkylgruppen, wie beispielsweise Benzyl oder Diphenylmethyl, heterocyclische Gruppen, wie Tetrahydropyrany und ähnliche, in Frage.

Als geeignete Thiolschutzgruppen kommen gegebenenfalls substituierte Alkylgruppen mit vorzugsweise 1 bis 6, insbesondere 1 bis 4 Kohlenstoffatomen, wie beispielsweise Acetamidomethyl und Chloracetamidomethyl, Phenylalkyl-haltige Alkylgruppen, wie beispielsweise Benzyl, 4-Methoxybenzyl, Diphenylmethyl, Triphenylmethyl und Pyridyl-diphenylmethyl und ähnliche, in Frage.

Die weiter obengenannten Schutzgruppen haben die in der Peptidchemie bekannte Funktion, Amino- Hydroxy- oder Thiolgruppen von Verbindungen temporär zu schützen.

Bevorzugt sind Verbindungen der allgemeinen Formel ( I ) und deren Salze 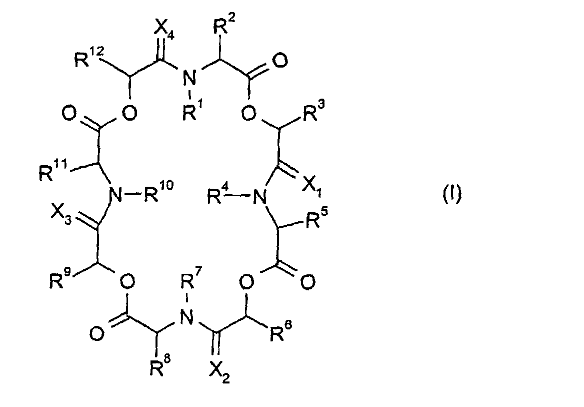 in welcher
- R¹, R⁴, R⁷ und R¹⁰: für C₁₋₄-Alkyl, insbesondere Methyl, stehen,
- R², R⁵, R⁸ und R¹¹: für C₁₋₄-Alkyl, insbesondere Isobutyl, stehen,
- R³ und R⁹: für C₁₋₄-Alkyl, insbesondere Methyl, stehen,
- R⁶ und R¹²: unabhängig voneinander für C₁₋₄-Alkyl, insbesondere Methyl, Hetaryl-C₁₋₂-alkyl, insbesondere Benzozhiazol-2-yl-methyl, Alkoxycarbonylmethyl, insbesondere Diphenylmethoxycarbonylmethyl und für gegebenenfalls substituiertes Benzyl, wobei als Substituenten genannt seien Wasserstoff, Halogen, insbesondere Brom, Fluor, Chlor oder Iod, Cyan, Carbamoyl, C₁₋₄-Alkyl, insbesondere Methyl, eine Schutzgruppe tragendes Hydroxy oder Hydroxy, C₁₋₈-Alkoxy, insbesondere Methoxy, Ethoxy, n-Propyloxy, Isopropyloxy, Butyloxy, tert-Butyloxy, Oktyloxy oder Triphenylmethoxy, C₁₋₄-Alkoxy-C₁₋₄-alkoxy, insbesondere 2-Methoxyethoxy, C₂₋₄-Alkenyloxy, insbesondere Allyloxy, Hetaryl-C₁₋₄-alkoxy, wobei die Heterocyclen wiederum mit Substituenten aus der Reihe C₁₋₄-Alkyl, insbesondere Methyl, Isopropyl, sec-Butyl und Isobutyl, C₃₋₆-Cycloalkyl, insbesondere Cyclopropyl und Cyclohexyl, Halogen, insbesondere Chlor, Brom, Fluor oder Iod, tragen können, insbesondere Fur-2-yl-methoxy, Fur-3-yl-methoxy, Tetrahydrofur-2-yl-methoxy, N-Boc-pyrrolidin-2-yl-methoxy, Pyrrolidin-2-yl-methoxy, 5-sec-Butyl-1,2,4-oxadiazol-3-yl-pyridyl-methoxy, 5-Cyclopropyl-1,2,4-oxadiazol-3-yl-methoxy, Imidazol-5-yl-methoxy, Thiazolyl-methoxy, Tetrazol-5-ylmethoxy, Nitro, eine Schutzgruppe tragendes Amino oder Amino, eine Schutzgruppe tragendes Amino-C₁₋₆-alkoxy oder Amino-C₁₋₆-alkoxy, insbesondere Aminoethoxy, N-Mono-C₁₋₆-alkylamino-C₁₋₆-alkoxy, insbesondere 2-N-Methylamino-ethoxy, N,N-Di-C₁₋₆-alkylamino-C₁₋₆-alkoxy, insbesondere N,N-Diethyl-amino-methoxy, 2-N,N-Dimethylamino-ethoxy oder 2-N,N-Diethylamino-ethoxy, N,N-Di-[(C₁₋₆-alkoxy-C₁₋₆-alkyl)]amino-C₁₋₆alkoxy, insbesondere 2-[N,N-Di(methoxyethyl)amino]-ethoxy, C₁₋₄-Alkylamino, insbesondere N-Methylamino, C₁₋₄-Dialkylamino, insbesondere Dimethylamino oder Diethylamino, C₃₋₇-Cycloalkylamino- (wobei als ringbildende Atome ein oder mehr Stickstoffatome enthalten und darüber hinaus Sauerstoff- oder Schwefelatome enthalten sein können) C₁₋₆-alkoxy, insbesondere 2-N-Morpholino-ethoxy, 2-N-Piperidino-ethoxy, Pyrrolidinomethoxy, C₃₋₇-Cyclo-alkylamino- (wobei als ringbildende Atome ein oder mehr Stickstoffatome enthalten und darüber hinaus Sauerstoff- oder Schwefelatome enthalten sein können), insbesondere N-Morpholino, N-Thiomorpholino, Piperidino, C₃₋₇-Cyclo-alkylamino- (wobei als ringbildende Atome ein oder mehr Stickstoffatome enthalten und darüber hinaus Sauerstoff- oder Schwefelatome enthalten sein können) sulfonyl, insbesondere N-Morpholino-sulfonyl, N-Thiomorpholino-sulfonyl, Piperidinosulfonyl, N-(N¹-Methyl-piperazino)-sulfonyl, N-(N¹-Methoxycarbonylpiperazino)-sulfonyl, N¹-Imidazolyl-sulfonyl, N,N-Di-[(C₁₋₆-alkoxy-C₁₋₆alkyl)]amino-sulfonyl, insbesondere 2-[N,N-Di(methoxyethyl)amino]-sulfonyl, C₃₋₇-Cyclo-alkylamino- (wobei als ringbildende Atome ein oder mehr Stickstoffatome enthalten und darüber hinaus Sauerstoff- oder Schwefelatome enthalten sein können)-C₁₋₆-alkylamino-sulfonyl, insbesondere 2-(N-Morpholino)-ethylamino-sulfonyl, C₁₋₄-Dialkylaminosulfonyl, insbesondere Diethylaminosulfonyl, Sulfamidyl,
und
(i)
   X¹ Schwefel bedeutet,
   X², X³ und X⁴ unabhängig voneinander Sauerstoff oder Schwefel bedeuten,
   oder
(ii)
   X² Schwefel bedeutet,
   X¹, X³ und X⁴ unabhängig voneinander Sauerstoff oder Schwefel bedeuten,
   oder
(iii)
   X³ Schwefel bedeutet,
   X¹, X² und X⁴ unabhängig voneinander Sauerstoff oder Schwefel bedeuten,
   oder
(iv)
   X⁴ Schwefel bedeutet,
   X¹, X² und X³ unabhängig voneinander Sauerstoff oder Schwefel bedeuten,
sowie deren optische Isomere und Racemate.

Besonders bevorzugt sind Verbindungen der allgemeinen Formel ( I ) und deren Salze 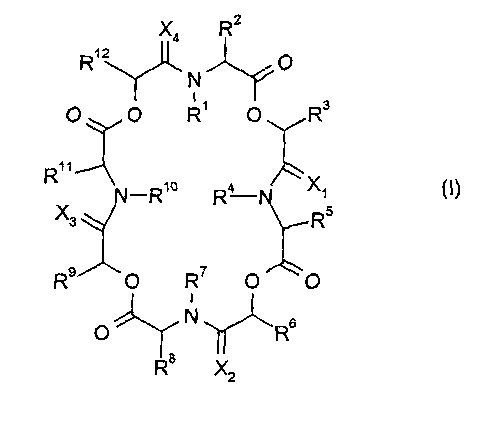 in welcher
(a) R¹, R⁴, R⁷ und R¹⁰ für Methyl stehen,
   R², R⁵, R⁸ und R¹¹ für Isobutyl stehen,
   R³ und R⁹ für Methyl stehen,
   R⁶ für unsubstituiertes Benzyl steht,
   R¹² für substituiertes Benzyl steht,
   wobei als Substituenten in para-Stellung des Phenylringes genannt seien Wasserstoff, C₁₋₈-Alkoxy, insbesondere Methoxy, tert-Butyloxy, Hetaryl-C₁₋₄alkoxy, insbesondere Fur-2-yl-methoxy, Tetrahydrofur-2-yl-methoxy, N-Bocpyrrolidin-2-yl-methoxy, Pyrid-2-yl-methoxy, Pyrrolidin-2-yl-methoxy, 5-sec-Butyl-1,2,4-oxadiazol-3-yl-methoxy, 5-Cyclopropyl-1,2,4-oxadiazol-3-yl-methoxy, Imidazol-5-yl-methoxy, Nitro, Amino, N,N-Di-C₁₋₆-alkylamino-C₁₋₆alkoxy, insbesondere N,N-Diethylamino-methoxy oder 2-N,N-Diethylaminoethoxy, N,N-Di-[(C₁₋₆-alkoxy-C₁₋₆-alkyl)]amino-C₁₋₆-alkoxy, insbesondere 2-[N,N-Di(methoxyethyl)amino]-ethoxy, C₁₋₄-Dialkylamino, insbesondere Dimethylamino, C₃₋₇-Cycloalkylamino- (wobei als ringbildende Atome ein oder mehr Stickstoffatome enthalten und darüber hinaus Sauerstoff- oder Schwefelatome enthalten sein können) -C₁₋₆-alkoxy, insbesondere 2-N-Morpholino-ethoxy, C₃₋₇-Cyclo-alkylamino-(wobei als ringbildende Atome ein oder mehr Stickstoffatome enthalten und darüber hinaus Sauerstoff- oder Schwefelatome enthalten sein können), insbesondere N-Morpholino, N-Thiomorpholino, Piperidino, C₃₋₇-Cycloalkylamino- (wobei als ringbildende Atome ein oder mehr Stickstoffatome enthalten und darüber hinaus Sauerstoff- oder Schwefelatome enthalten sein können) -sulfonyl, insbesondere N-Morpholino-sulfonyl, Piperidinosulfonyl, N-(N¹-Methoxycarbonyl-piperazino)-sulfonyl, N,N-Di-[(C₁₋₆-alkoxy-C₁₋₆-alkyl)]amino-sulfonyl, insbesondere 2-[N,N-Di(methoxyethyl)amino]-sulfonyl, C₁₋₄-Dialkylamino-sulfonyl, insbesondere Diethylaminosulfonyl,
   und
   (i)
      X¹ Schwefel bedeutet,
      X², X³ und X⁴ unabhängig voneinander Sauerstoff oder Schwefel bedeuten,
      oder
   (ii)
      X² Schwefel bedeutet,
      X¹, X³ und X⁴ unabhängig voneinander Sauerstoff oder Schwefel bedeuten,
      oder
   (iii)
      X³ Schwefel bedeutet,
      X¹, X² und X⁴ unabhängig voneinander Sauerstoff oder Schwefel bedeuten,
      oder
   (iv)
      X⁴ Schwefel bedeutet,
      X¹, X² und X³ unabhängig voneinander Sauerstoff oder Schwefel bedeuten,
      oder
(b) R¹, R⁴, R⁷ und R¹⁰ für Methyl stehen,
   R², R⁵, R⁸ und R¹¹ für Isobutyl stehen,
   R³ und R⁹ für Methyl stehen,
   R⁶ und R¹² für jeweils gleich substituiertes Benzyl stehen,
   wobei als Substituenten in para-Stellung des Phenylringes genannt seien Wasserstoff, C₁₋₈-Alkoxy, insbesondere Methoxy, tert-Butyloxy, Hetaryl-C₁₋₄alkoxy, insbesondere Fur-2-yl-methoxy, Tetrahydrofur-2-yl-methoxy, N-Bocpyrrolidin-2-yl-methoxy, Pyrid-2-yl-methoxy, Pyrrolidin-2-yl-methoxy, 5-sec-Butyl-1,2,4-oxadiazol-3-yl-methoxy, 5-Cyclopropyl-1,2,4-oxadiazol-3-ylmethoxy, Imidazol-5-yl-methoxy, Nitro, Amino, N,N-Di-C₁₋₆-alkylamino-C₁₋₆alkoxy, insbesondere N,N-Diethylamino-methoxy oder 2-N,N-Diethylaminoethoxy, N,N-Di-[(C₁₋₆-alkoxy-C₁₋₆-alkyl)]amino-C₁₋₆-alkoxy, insbesondere 2-[N,N-Di(methoxyethyl)amino]-ethoxy, C₁₋₄-Dialkylamino, insbesondere Dimethylamino, C₃₋₇-Cycloalkylamino- (wobei als ringbildende Atome ein oder mehr Stickstoffatome enthalten und darüber hinaus Sauerstoff- oder Schwefelatome enthalten sein können) -C₁₋₆-alkoxy, insbesondere 2-N-Morpholino-ethoxy, C₃₋₇-Cyclo-alkylamino- (wobei als ringbildende Atome ein oder mehr Stickstoffatome enthalten und darüber hinaus Sauerstoffoder Schwefelatome enthalten sein können), insbesondere N-Morpholino, N-Thiomorpholino, Piperidino, C₃₋₇-Cycloalkylamino- (wobei als ringbildende Atome ein oder mehr Stickstoffatome enthalten und darüber hinaus Sauerstoff- oder Schwefelatome enthalten sein können) -sulfonyl, insbesondere N-Morpholino-sulfonyl, Piperidinosulfonyl, N-(N¹-Methoxycarbonyl-piperazino)-sulfonyl, N,N-Di-[(C₁₋₆-alkoxy-C₁₋₆-alkyl)]amino-sulfonyl, insbesondere 2-[N,N-Di(methoxyethyl)amino]-sulfonyl, C₁₋₄-Dialkylamino-sulfonyl, insbesondere Diethylaminosulfonyl,
   und
   (i)
      X¹ Schwefel bedeutet,
      X², X³ und X⁴ unabhängig voneinander Sauerstoff oder Schwefel bedeuten,
      oder
   (ii)
      X² Schwefel bedeutet,
      X¹, X³ und X⁴ unabhängig voneinander Sauerstoff oder Schwefel bedeuten,
      oder
   (iii)
      X³ Schwefel bedeutet,
      X¹, X² und X⁴ unabhängig voneinander Sauerstoff oder Schwefel bedeuten,
      oder
   (iv)
      X⁴ Schwefel bedeutet,
      X¹, X² und X³ unabhängig voneinander Sauerstoff oder Schwefel bedeuten,
   sowie deren optische Isomere und Racemate.

Insbesondere bevorzugt sind Verbindungen der allgemeinen Formel (I) und deren Salze 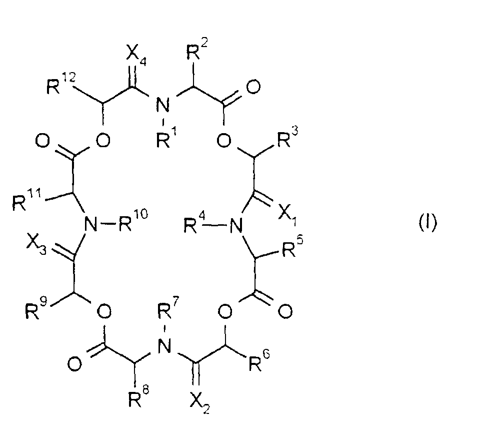 in welcher
(a) R¹, R⁴, R⁷ und R¹⁰ für Methyl stehen,
   R², R⁵, R⁸ und R¹¹ für Isobutyl stehen,
   R³ und R⁹ für Methyl stehen,
   R⁶ für unsubstituiertes Benzyl steht,
   R¹² für substituiertes Benzyl steht,
   wobei als Substituenten in para-Stellung des Phenylringes genannt seien Wasserstoff, C₁₋₈-Alkoxy, insbesondere tert-Butyloxy, Hetaryl-C₁₋₄-alkoxy, insbesondere Fur-2-yl-methoxy, Tetrahydrofur-2-yl-methoxy, N-Boc-pyrrolidin-2-yl-methoxy, Pyrid-2-yl-methoxy, Pyrrolidin-2-yl-methoxy, 5-sec-Butyl-1,2,4-oxadiazol-3-yl-methoxy, Nitro, Amino, N,N-Di-C₁₋₆-alkylamino-C₁₋₆alkoxy, insbesondere 2-N,N-Diethylamino-ethoxy, N,N-Di-[(C₁₋₆-alkoxy-C₁₋₆alkyl)]amino-C₁₋₆-alkoxy, insbesondere 2-[N,N-Di(methoxyethyl)amino]-ethoxy, C₁₋₄-Dialkylamino, insbesondere Dimethylamino, C₃₋₇-Cyclo-alkylamino- (wobei als ringbildende Atome ein oder mehr Stickstoffatome enthalten und darüber hinaus Sauerstoff- oder Schwefelatome enthalten sein können) -C₁₋₆-alkoxy, insbesondere 2-N-Morpholino-ethoxy, C₃₋₇-Cycloalkylamino- (wobei als ringbildende Atome ein oder mehr Stickstoffatome enthalten und darüber hinaus Sauerstoff- oder Schwefelatome enthalten sein können), insbesondere N-Morpholino,
   und
   (i)
      X¹ Schwefel bedeutet,
      X², X³ und X⁴ unabhängig voneinander Sauerstoff oder Schwefel bedeuten,
      oder
   (ii)
      X² Schwefel bedeutet,
      X¹, X³ und X⁴ unabhängig voneinander Sauerstoff oder Schwefel bedeuten,
      oder
   (iii)
      X³ Schwefel bedeutet,
      X¹, X² und X⁴ unabhängig voneinander Sauerstoff oder Schwefel bedeuten,
      oder
   (iv)
      X⁴ Schwefel bedeutet,
      X¹, X² und X³ unabhängig voneinander Sauerstoff oder Schwefel bedeuten,
      oder
(b) R¹, R⁴, R⁷ und R¹⁰ für Methyl stehen,
   R², R⁵, R⁸ und R¹¹ für Isobutyl stehen,
   R³ und R⁹ für Methyl stehen,
   R⁶ und R¹² für jeweils gleich substituiertes Benzyl stehen,
   wobei als Substituenten in para-Stellung des Phenylringes genannt seien Wasserstoff, C₁₋₈-Alkoxy, insbesondere tert-Butyloxy, Hetaryl-C₁₋₄-alkoxy, insbesondere Fur-2-yl-methoxy, Tetrahydrofur-2-yl-methoxy, N-Boc-pyrrolidin-2-yl-methoxy, Pyrid-2-yl-methoxy, Pyrrolidin-2-yl-methoxy, 5-sec-Butyl-1,2,4-oxadiazol-3-yl-methoxy, Nitro, Amino, N,N-Di-C₁₋₆-alkylamino-C₁₋₆alkoxy, insbesondere 2-N,N-Diethylaminoethoxy, N,N-Di-[(C₁₋₆-alkoxy-C₁₋₆alkyl)]amino-C₁₋₆-alkoxy, insbesondere 2-[N,N-Di(methoxyethyl)amino]-ethoxy, C₁₋₄-Dialkylamino, insbesondere Dimethylamino, C₃₋₇-Cyclo-alkylamino- (wobei als ringbildende Atome ein oder mehr Stickstoffatome enthalten und darüber hinaus Sauerstoff- oder Schwefelatome enthalten sein können) -C₁₋₆-alkoxy, insbesondere 2-N-Morpholino-ethoxy, C₃₋₇-Cycloalkylamino- (wobei als ringbildende Atome ein oder mehr Stickstoffatome enthalten und darüber hinaus Sauerstoff- oder Schwefelatome enthalten sein können), insbesondere N-Morpholino,
   und
   (i)
      X¹ Schwefel bedeutet,
      X², X³ und X⁴ unabhängig voneinander Sauerstoff oder Schwefel bedeuten,
      oder
   (ii)
      X² Schwefel bedeutet,
      X¹, X³ und X⁴ unabhängig voneinander Sauerstoff oder Schwefel bedeuten,
      oder
   (iii)
      X³ Schwefel bedeutet,
      X¹, X² und X⁴ unabhängig voneinander Sauerstoff oder Schwefel bedeuten,
      oder
   (iv)
      X⁴ Schwefel bedeutet,
      X¹, X² und X³ unabhängig voneinander Sauerstoff oder Schwefel bedeuten,

Die erfindungsgemäß zu verwendenden Thiodepsipeptide der allgemeinen Formel (I) und deren Salze enthalten außerdem ein oder mehrere Chiralitätszentren und können somit als reine Stereoisomere oder in Form verschiedener Enantiomeren- und Diastereomerengemische vorliegen, die erforderlichenfalls in an sich bekannter Weise getrennt werden können oder auch durch stereoselektive Reaktionen in Kombination mit dem Einsatz von stereochemisch reinen Ausgangsstoffen hergestellt werden.

Vorzugsweise werden jedoch die optisch aktiven, stereoisomeren Formen der Verbindungen der allgemeinen Formel ( I ) und deren Salze erfindungsgemäß verwendet. Besonders bevorzugt werden die cyclischen Depsipeptide verwendet, die sich aus (S)-konfigurierten Aminosäuren (L-Form) und (R)-konfigurierten Hydroxycarbonsäuren (D-Form) als Ringbausteine zusammensetzen.

Die Erfindung betrifft daher sowohl die reinen Enantiomeren und Diastereomeren, als auch deren Gemische zur Bekämpfung von Endoparasiten, besonders auf dem Gebiet der Medizin und Veterinärmedizin.

Als geeignete Salze der Thiodepsipeptide der allgemeinen Formel (I) können übliche nicht toxische Salze, d.h. Salze mit verschiedenen Basen und Salze mit zugesetzten Säuren, genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calzium- oder Magnesiumsalze, Ammoniumsalze, Salze mit organischen Basen sowie mit anorganischen Aminen, beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren, beispielsweise Hydrochloride, Hydrobromide, Dihydrosulfate, Trihydrosulfate, oder Phosphate, Salze mit organischen Carbonsäuren oder organischen Sulfosäuren, beispielsweise Formiate, Acetate, Trifluoracetate, Maleate, Tartrate, Methansulfonate, Benzolsulfonate oder para-Toluolsulfonate, Salze mit basischen Aminosäuren, beispielsweise Arginate, Aspartate oder Glutamate und Änliches zu nennen.

Im einzelnen seien die nachfolgenden Cyclodepsipeptide mit 24 Ringatomen genannt:
Cyclo(-N-methyl-L-leucinyl-D-thiolactyl-N-methyl-L-leucinyl-D-phenylthiolactyl-N-methyl-L-leucinyl-D-thiolactyl-N-methyl-L-leucinyl-D-phenylthiolactyl-),
Cyclo(-N-methyl-L-leucinyl-D-lactyl-N-methyl-L-leucinyl-D-phenylthiolactyl-N-methyl-L-leucinyl-D-thiolactyl-N-methyl-L-leucinyl-D-phenylthiolactyl-),
Cyclo(-N-methyl-L-leucinyl-D-thiolactyl-N-methyl-L-leucinyl-D-phenylthiolactyl-N-methyl-L-leucinyl-D-thiolactyl-N-methyl-L-leucinyl-D-phenyllactyl-),
Cyclo(-N-methyl-L-leucinyl-D-thiolactyl-N-methyl-L-leucinyl-D-phenylthiolactyl-N-methyl-L-leucinyl-D-lactyl-N-methyl-L-leucinyl-D-phenyllactyl-),
Cyclo(-N-methyl-L-leucinyl-D-lactyl-N-methyl-L-leucinyl-D-phenylthiolactyl-N-methyl-L-leucinyl-D-thiolactyl-N-methyl-L-leucinyl-D-phenyllactyl-),
Cyclo(-N-methyl-L-leucinyl-D-thiolactyl-N-methyl-L-leucinyl-D-phenyllactyl-N-methyl-L-leucinyl-D-thiolactyl-N-methyl-L-leucinyl-D-phenyllactyl-),
Cyclo(-N-methyl-L-leucinyl-D-lactyl-N-methyl-L-leucinyl-D-phenylthiolactyl-N-methyl-L-leucinyl-D-lactyl-N-methyl-L-leucinyl-D-phenylthiolactyl-),
Cyclo(-N-methyl-L-leucinyl-D-thiolactyl-N-methyl-L-leucinyl-D-phenyllactyl-N-methyl-L-leucinyl-D-lactyl-N-methyl-L-leucinyl-D-phenyllactyl-),
Cyclo(-N-methyl-L-leucinyl-D-lactyl-N-methyl-L-leucinyl-D-phenylthiolactyl-N-methyl-L-leucinyl-D-lactyl-N-methyl-L-leucinyl-D-phenyllactyl-),
Cyclo(-N-methyl-L-leucinyl-D-thiolactyl-N-methyl-L-leucinyl-D-4-nitro-phenylthiolactyl-N-methyl-L-leucinyl-D-thiolactyl-N-methyl-L-leucinyl-D-4-vitro-phenylthiolactyl-),
Cyclo(-N-methyl-L-leucinyl-D-thiolactyl-N-methyl-L-leucinyl-D-4-nitro-phenyllactyl-N-methyl-L-leucinyl-D-lactyl-N-methyl-L-leucinyl-D-4-nitro-phenyllactyl-),
Cyclo(-N-methyl-L-leucinyl-D-thiolactyl-N-methyl-L-leucinyl-D-4-tert-butyloxyphenylthiolactyl-N-methyl-L-leucinyl-D-thiolactyl-N-methyl-L-leucinyl-D-4-tertbutyloxy-phenylthiolactyl-),
Cyclo(-N-methyl-L-leucinyl-D-thiolactyl-N-methyl-L-leucinyl-D-4-tert-butyloxyphenyllactyl-N-methyl-L-leucinyl-D-lactyl-N-methyl-L-leucinyl-D-4-tert-butyloxyphenyllactyl-),
Cyclo(-N-methyl-L-leucinyl-D-thiolactyl-N-methyl-L-leucinyl-D-4-N-morpholinophenylthiolactyl-N-methyl-L-leucinyl-D-thiolactyl-N-methyl-L-leucinyl-D-4-N-morpholino-phenylthiolactyl-),
Cyclo(-N-methyl-L-leucinyl-D-lactyl-N-methyl-L-leucinyl-D-4-N-morpholino-phenylthiolactyl-N-methyl-L-leucinyl-D-thiolactyl-N-methyl-L-leucinyl-D-4-N-morpholinophenyl-thiolactyl-),
Cyclo(-N-methyl-L-leucinyl-D-thiolactyl-N-methyl-L-leucinyl-D-4-N-morpholinophenylthiolactyl-N-methyl-L-leucinyl-D-thiolactyl-N-methyl-L-leucinyl-D-4-N-morpholino-phenyllactyl-),
Cyclo(-N-methyl-L-leucinyl-D-thiolactyl-N-methyl-L-leucinyl-D-4-N-morpholinophenylthiolactyl-N-methyl-L-leucinyl-D-lactyl-N-methyl-L-leucinyl-D-4-N-morpholino-phenyllactyl-),
Cyclo(-N-methyl-L-leucinyl-D-lactyl-N-methyl-L-leucinyl-D-4-N-morpholino-phenylthiolactyl-N-methyl-L-leucinyl-D-thiolactyl-N-methyl-L-leucinyl-D-4-N-morpholinophenyllactyl-),
Cyclo(-N-methyl-L-leucinyl-D-thiolactyl-N-methyl-L-leucinyl-D-4-N-morpholinophenyllactyl-N-methyl-L-leucinyl-D-thiolactyl-N-methyl-L-leucinyl-D-4-N-morpholino-phenyllactyl-),
Cyclo(-N-methyl-L-leucinyl-D-lactyl-N-methyl-L-leucinyl-D-4-N-morpholino-phenylthiolactyl-N-methyl-L-leucinyl-D-lactyl-N-methyl-L-leucinyl-D-4-N-morpholino-phenylthiolactyl-),
Cyclo(-N-methyl-L-leucinyl-D-thiolactyl-N-methyl-L-leucinyl-D-4-N-morpholinophenyllactyl-N-methyl-L-leucinyl-D-lactyl-N-methyl-L-leucinyl-D-4-N-morpholinophenyllactyl-),
Cyclo(-N-methyl-L-leucinyl-D-lactyl-N-methyl-L-leucinyl-D-4-N-morpholino-phenylthiolactyl-N-methyl-L-leucinyl-D-lactyl-N-methyl-L-leucinyl-D-4-N-morpholinophenyllactyl-),
Cyclo[-N-methyl-L-leucinyl-D-thiolactyl-N-methyl-L-leucinyl-D-4-(fur-2-yl-methoxy)-phenylthiolactyl-N-methyl-L-leucinyl-D-thiolactyl-N-methyl-L-leucinyl-D-4-(fur-2-yl-methoxy)-phenylthiolactyl-],
Cyclo[-N-methyl-L-leucinyl-D-thiolactyl-N-methyl-L-leucinyl-D-4-(fur-2-yl-methoxy)-phenyllactyl-N-methyl-L-leucinyl-D-thiolactyl-N-methyl-L-leucinyl-D-4-(fur-2-yl-methoxy)-phenyllactyl-],
Cyclo[-N-methyl-L-leucinyl-D-thiolactyl-N-methyl-L-leucinyl-D-4-(fur-2-yl-methoxy)-phenyllactyl-N-methyl-L-leucinyl-D-lactyl-N-methyl-L-leucinyl-D-4-(fur-2-ylmethoxy)-phenyllactyl-],
Cyclo[-N-methyl-L-leucinyl-D-thiolactyl-N-methyl-L-leucinyl-D-4-(fur-2-yl-methoxy)-phenylthiolactyl-N-methyl-L-leucinyl-D-thiolactyl-N-methyl-L-leucinyl-Dphenylthio-lactyl-],
Cyclo[-N-methyl-L-leucinyl-D-thiolactyl-N-methyl-L-leucinyl-D-4-(fur-2-yl-methoxy)-phenyllactyl-N-methyl-L-leucinyl-D-thiolactyl-N-methyl-L-leucinyl-D-phenyllactyl-],
Cyclo[-N-methyl-L-leucinyl-D-thiolactyl-N-methyl-L-leucinyl-D-4-(fur-2-yl-methoxy)-phenyllactyl-N-methyl-L-leucinyl-D-lactyl-N-methyl-L-leucinyl-D-phenyllactyl-],
Cyclo[-N-methyl-L-leucinyl-D-lactyl-N-methyl-L-leucinyl-D-4-(fur-2-yl-methoxy)-phenyllactyl-N-methyl-L-leucinyl-D-thiolactyl-N-methyl-L-leucinyl-D-phenyllactyl-],
Cyclo[-N-methyl-L-leucinyl-D-thiolactyl-N-methyl-L-leucinyl-D-4-(tetrahydrofur-2-yl-methoxy)-phenylthiolactyl-N-methyl-L-leucinyl-D-thiolactyl-N-methyl-L-leucinyl-D-4-(tetrahydrofur-2-yl-methoxy)-phenylthiolactyl-],
Cyclo[-N-methyl-L-leucinyl-D-thiolactyl-N-methyl-L-leucinyl-D-4-(tetrahydrofur-2-yl-methoxy)-phenyllactyl-N-methyl-L-leucinyl-D-thiolactyl-N-methyl-L-leucinyl-D-4-(tetrahydrofur-2-yl-methoxy)-phenyllactyl-],
Cyclo[-N-methyl-L-leucinyl-D-thiolactyl-N-methyl-L-leucinyl-D-4-(tetrahydrofur-2-yl-methoxy)-phenyllactyl-N-methyl-L-leucinyl-D-lactyl-N-methyl-L-leucinyl-D-4-(tetra-hydrofur-2-yl-methoxy)-phenyllactyl-],
Cyclo[-N-methyl-L-leucinyl-D-thiolactyl-N-methyl-L-leucinyl-D-4-(pyrrolidin-2-ylmethoxy)-phenylthiolactyl-N-methyl-L-leucinyl-D-thiolactyl-N-methyl-L-leucinyl-D-4-(pyrrolidin-2-yl-methoxy)-phenylthiolactyl-],
Cyclo[-N-methyl-L-leucinyl-D-thiolactyl-N-methyl-L-leucinyl-D-4-(pyrrolidin-2-ylmethoxy)-phenyl(actyl-N-methyl-L-leucinyl-D-thiolactyl-N-methyl-L-leucinyl-D-4-(pyrrolidin-2-yl-methoxy)-phenyllactyl-],
Cyclo[-N-methyl-L-leucinyl-D-thiolactyl-N-methyl-L-leucinyl-D-4-(pyrrolidin-2-ylmethoxy)-phenyllactyl-N-methyl-L-leucinyl-D-lactyl-N-methyl-L-leucinyl-D-4-(pyrrolidin-2-yl-methoxy)-phenyllactyl-],
Cyclo[-N-methyl-L-leucinyl-D-thiolactyl-N-methyl-L-leucinyl-D-4-(pyrrolidin-2-ylmethoxy)-phenylthiolactyl-N-methyl-L-leucinyl-D-thiolactyl-N-methyl-L-leucinyl-Dphenylthiolactyl-],
Cyclo[-N-methyl-L-leucinyl-D-thiolactyl-N-methyl-L-leucinyl-D-4-(pyrrolidin-2-ylmethoxy)-phenyl(actyl-N-methyl-L-leucinyl-D-thiolactyl-N-methyl-L-leucinyl-Dphenyllactyl-],
Cyclo[-N-methyl-L-leucinyl-D-thiolactyl-N-methyl-L-leucinyl-D-4-(pyrrolidin-2-ylmethoxy)-phenyllactyl-N-methyl-L-leucinyl-D-lactyl-N-methyl-L-leucinyl-D-phenyllactyl-],
Cyclo[-N-methyl-L-leucinyl-D-lactyl-N-methyl-L-leucinyl-D-4-(pyrrolidin-2-yl-methoxy)-phenyllactyl-N-methyl-L-leucinyl-D-thiolactyl-N-methyl-L-leucinyl-D-phenyllactyl-],
Cyclo[-N-methyl-L-leucinyl-D-thiolactyl-N-methyl-L-leucinyl-D-4-(5-sec-butyl-1,2,4-oxadiazol-3-yl-methoxy)-phenylthiolactyl-N-methyl-L-leucinyl-D-thiolactyl-N-methyl-L-leucinyl-D-4-(5-sec-butyl-1,2,4-oxadiazol-3-yl-methoxy)-phenylthiolactyl-],
Cyclo[-N-methyl-L-leucinyl-D-thiolactyl-N-methyl-L-leucinyl-D-4-(5-sec-butyl-1,2,4-oxadiazol-3-yl-methoxy)-phenyllactyl-N-methyl-L-leucinyl-D-thiolactyl-N-methyl-Lleucinyl-D-4-(5-sec-butyl-1,2,4-oxadiazol-3-yl-methoxy)-phenyllactyl-],
Cyclo[-N-methyl-L-leucinyl-D-thiolactyl-N-methyl-L-leucinyl-D-4-(5-sec-butyl-1,2,4-oxadiazol-3-yl-methoxy)-phenyllactyl-N-methyl-L-leucinyl-D-lactyl-N-methyl-L-leucinyl-D-4-(5-sec-butyl-1,2,4-oxadiazol-3-yl-methoxy)-phenyllactyl-],
Cyclo[-N-methyl-L-leucinyl-D-thiolactyl-N-methyl-L-leucinyl-D-4-(5-sec-butyl-1,2,4-oxadiazol-3-yl-methoxy)-phenylthiolactyl-N-methyl-L-leucinyl-D-thiolactyl-N-methyl-L-leucinyl-D-phenylthiolactyl-],
Cyclo[-N-methyl-L-leucinyl-D-thiolactyl-N-methyl-L-leucinyl-D-4-(5-cyclopropyl-1,2,4-oxadiazol-3-yl-methoxy)-phenylthiolactyl-N-methyl-L-leucinyl-D-thiolactyl-N-methyl-L-leucinyl-D-phenylthiolactyl-],
Cyclo[-N-methyl-L-leucinyl-D-thiolactyl-N-methyl-L-leucinyl-D-4-(tetrazol-5-yl-methoxy)-phenylthiolactyl-N-methyl-L-leucinyl-D-thiolactyl-N-methyl-L-leucinyl-Dphenyl-thiolactyl-],
Cyclo[-N-methyl-L-leucinyl-D-thiolactyl-N-methyl-L-leucinyl-D-4-(2-N,N-diethylamino-ethoxy)-phenylthiolactyl-N-methyl-L-leucinyl-D-thiolactyl-N-methyl-L-leucinyl-D-phenylthiolactyl-],
Cyclo[-N-methyl-L-leucinyl-D-thiolactyl-N-methyl-L-leucinyl-D-4-(2-N,N-diethylamino-ethoxy)-phenylthiolactyl-N-methyl-L-leucinyl-D-thiolactyl-N-methyl-L-leucinyl-D-4-(2-N,N-diethylamino-ethoxy)-phenylthiolactyl-],
Cyclo[-N-methyl-L-leucinyl-D-thiolactyl-N-methyl-L-leucinyl-D-4-(2-N,N-diethylamino-ethoxy)-phenyllactyl-N-methyl-L-leucinyl-D-thiolactyl-N-methyl-L-leucinyl-D-4-(2-N, N-diethylamino-ethoxy)-phenyllactyl-],
Cyclo[-N-methyl-L-leucinyl-D-thiolactyl-N-methyl-L-leucinyl-D-4-(2-N,N-diethylamino-ethoxy)-phenyllactyl-N-methyl-L-leucinyl-D-lactyl-N-methyl-L-leucinyl-D-4-(2-N,N-di-ethylamino-ethoxy)-phenyllactyl-],
Cyclo[-N-methyl-L-leucinyl-D-thiolactyl-N-methyl-L-leucinyl-D-4-(N,N-diethylamino-methoxy)-phenylthiolactyl-N-methyl-L-leucinyl-D-thiolactyl-N-methyl-L-leucinyl-D-4-(N,N-diethylaminomethoxy)-phenylthiolactyl-],
Cyclo[-N-methyl-L-leucinyl-D-thiolactyl-N-methyl-L-leucinyl-D-4-(N,N-diethylamino-methoxy)-phenyllactyl-N-methyl-L-leucinyl-D-thiolactyl-N-methyl-L-leucinyl-D-4-(N,N-diethylaminomethoxy)-phenyllactyl-],
Cyclo[-N-methyl-L-leucinyl-D-thiolactyl-N-methyl-L-leucinyl-D-4-(N,N-diethylamino-methoxy)-phenyllactyl-N-methyl-L-leucinyl-D-lactyl-N-methyl-L-leucinyl-D-4-(N,N-di-ethylaminomethoxy)-phenyllactyl-],
Cyclo[-N-methyl-L-leucinyl-D-thiotactyl-N-methyl-L-leucinyl-D-4-(2-N-morpholinoethoxy)-phenylthiolactyl-N-methyl-L-leucinyl-D-thiolactyl-N-methyl-L-leucinyl-D-4-(2-N-morpholino-ethoxy)-phenylthiolactyl-],
Cyclo[-N-methyl-L-leucinyl-D-thiolactyl-N-methyl-L-leucinyl-D-4-(2-N-morpholinoethoxy)-phenyllactyl-N-methyl-L-leucinyl-D-lactyl-N-methyl-L-leucinyl-D-4-(2-N-morpholino-ethoxy)-phenyllactyl-],
Cyclo[-N-methyl-L-leucinyl-D-thiolactyl-N-methyl-L-leucinyl-D-4-(N,N-dimethoxyethylamino-ethoxy)-phenylthiolactyl-N-methyl-L-leucinyl-D-thiolactyl-N-methyl-Lleucinyl-D-4-(N,N-dimethoxy-ethylamino-ethoxy)-phenylthiolactyl-],
Cyclo[-N-methyl-L-leucinyl-D-thiolactyl-N-methyl-L-leucinyl-D-4-(N,N-dimethoxyethylamino-ethoxy)-phenylthiolactyl-N-methyl-L-leucinyl-D-thiolactyl-N-methyl-Lleucinyl-D-phenylthiolactyl-],
Cyclo[-N-methyl-L-leucinyl-D-thiolactyl-N-methyl-L-leucinyl-D-4-(N-piperidinosulfonyl)-phenylthiolactyl-N-methyl-L-leucinyl-D-thiolactyl-N-methyl-L-leucinyl-D-4-(N-pi-peridino-sulfonyl)-phenylthiolactyl-],
Cyclo[-N-methyl-L-leucinyl-D-thiolactyl-N-methyl-L-leucinyl-D-4-(N-piperidinosulfonyl)-phenyllactyl-N-methyl-L-leucinyl-D-lactyl-N-methyl-L-leucinyl-D-4-(Npiperidino-sulfonyl)-phenyllactyl-],
Cyclo[-N-methyl-L-leucinyl-D-thiolactyl-N-methyl-L-leucinyl-D-4-(N-morpholinomethoxy)-phenylthiolactyl-N-methyl-L-leucinyl-D-thiolactyl-N-methyl-L-leucinyl-Dphenylthiolactyl-],
Cyclo[-N-methyl-L-leucinyl-D-thiolactyl-N-methyl-L-leucinyl-D-4-(N-morpholinomethoxy)-phenyllactyl-N-methyl-L-leucinyl-D-lactyl-N-methyl-L-leucinyl-D-phenyllactyl-],
Cyclo[-N-methyl-L-leucinyl-D-thiolactyl-N-methyl-L-leucinyl-D-4-(N,N-diethylamino-methoxy)-phenylthiolactyl-N-methyl-L-leucinyl-D-thiolactyl-N-methyl-L-leucinyl-D-phenylthiolactyl-],
Cyclo[-N-methyl-L-leucinyl-D-thiolactyl-N-methyl-L-leucinyl-D-4-(N,N-diethylamino-methoxy)-phenyllactyl-N-methyl-L-leucinyl-D-lactyl-N-methyl-L-leucinyl-Dphenyllactyl-],
Cyclo[-N-methyl-L-leucinyl-D-thiolactyl-N-methyl-L-leucinyl-D-4-(pyrid-2-yl-methoxy)-phenylthiolactyl-N-methyl-L-leucinyl-D-thiolactyl-N-methyl-L-leucinyl-D-4-(pyrid-2-yl-methoxy)-phenylthiolactyl-],
Cyclo[-N-methyl-L-leucinyl-D-thiolactyl-N-methyl-L-leucinyl-D-4-(pyrid-2-yl-methoxy)-phenyllactyl-N-methyl-L-leucinyl-D-lactyl-N-methyl-L-leucinyl-D-4-(pyrid-2-yl-methoxy)-phenyllactyl-],
Cyclo[-N-methyl-L-leucinyl-D-thiolactyl-N-methyl-L-leucinyl-D-4-(pyrid-2-yl-methoxy)-phenyllactyl-N-methyl-L-leucinyl-D-thiolactyl-N-methyl-L-leucinyl-D-4-(pyrid-2-yl-methoxy)-phenyllactyl-].

Die Verbindungen der allgemeinen Formel (I) sind neu, sie lassen sich beispielsweise nach dem oben angegebenen Verfahren herstellen.

Überraschenderweise können die neuen Thiodepsipeptide der allgemeinen Formel (I) durch Thionierung einer oder mehrerer Amidgruppen aus entsprechenden Depsipeptiden der allgemeinen Formel (II) erhalten werden.

Nachfolgend wird das erfindungsgemäße Verfahren anhand ausgewählter Bei-spiele erläutert (vgl. auch Herstellungsbeispiele).

Setzt man beispielsweise bei Verfahren 2 zur Thionierung das cyclische Depsipeptid Cyclo(-N-methyl-L-leucinyl-D-lactyl-N-methyl-L-leucinyl-D-phenyllactyl-N-methyl-L-leucinyl-D-lactyl-N-methyl-L-leucinyl-D-phenyllactyl-) (PF 1022A) als Verbindung der allgemeinen Formel (II) ein und verwendet als Thionierungsmittel *"Lawesson's Reagens"*, so entsteht das Cyclo(-N-methyl-L-leucinyl-D-thiolactyl-N-methyl-L-leucinyl-D-phenylthiolactyl-N-methyl-L-leucinyl-D-thiolactyl-N-methyl-L-leucinyl-Dphenyl-thiolactyl-) (vgl. Schema I).

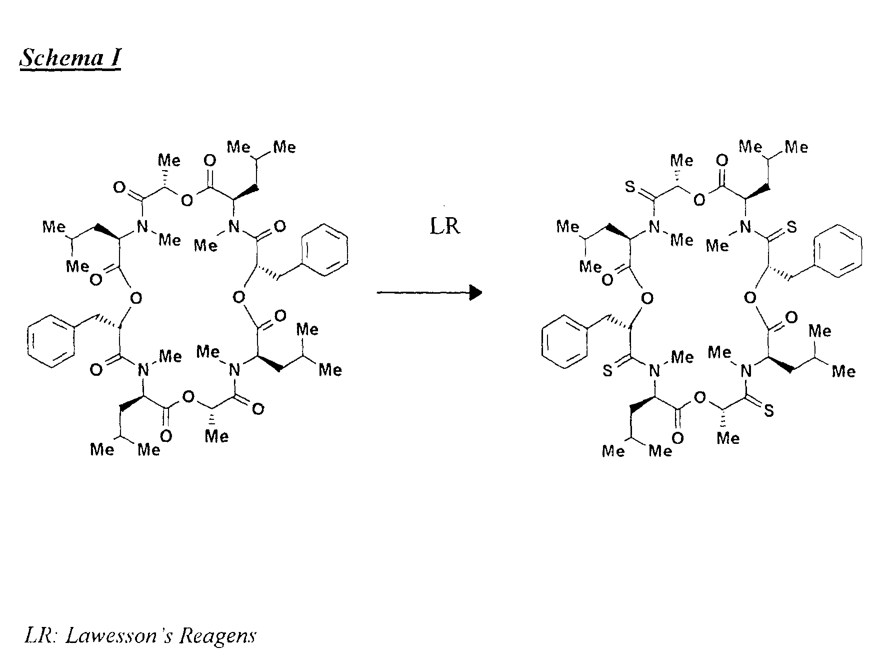

Setzt man in einer weiteren Ausführungsform des Verfahrens 2 zur Thionierung das cyclische Depsipeptid Cyclo(-N-methyl-L-leucinyl-D-lactyl-N-methyl-L-leucinyl-Dphenyllactyl-N-methyl-L-leucinyl-D-lactyl-N-methyl-L-leucinyl-D-phenyllactyl-) (PF 1022A) als Verbindung der allgemeinen Formel (II) ein und verwendet als Thionierungsmittel *"Belleau's Reagens"*, so entsteht selektiv das Cyclo(-N-methyl-L-leucinyl-D-thiolactyl-N-methyl-L-leucinyl-D-phenyllactyl-N-methyl-L-leueinyl-D-lactyl-N-methyl-L-leucinyl-D-phenyllactyl-) (vgl. Schema II).

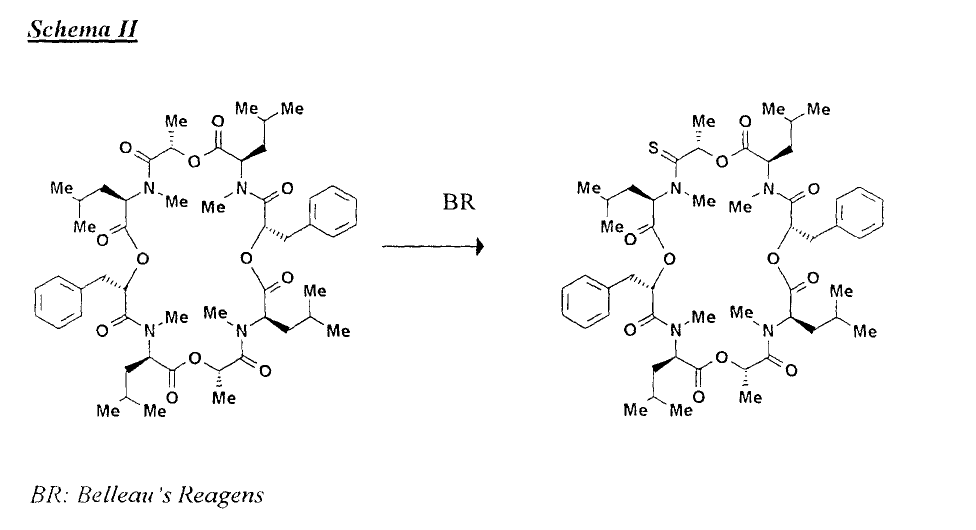

Die zur Durchführung des Verfahrens als Ausgangsstoffe benötigten Verbindungen sind durch die Formeln (II) allgemein definiert. In den Formeln stehen vorzugsweise für R¹ bis R¹² diejenigen Reste, die bereits im Zusammenhang mit der Beschreibung der erfindungsgemäßen Stoffe der Formel (I) als bevorzugt für diese Substituenten genannt wurden.

Die als Ausgangsstoffe verwendeten cyclischen Depsipeptide sind z. T. bekannt und können nach literaturbekannten Methoden mittels Fermentation oder Totalsynthese erhalten werden [vgl. Fermentation der Cyclooktadepsipeptide: PF 1022A: Fermentation aus *Mycelia sterilia* (FERM BP-2671; frühere Bezeichnung FERM P-10 504) in EP-OS 382 173; T. Sasaki et al., J. Antibiotics 45, 1992, S. 692; US-Pat. 5 116 815; EP-OS 503 538; Y. Kodama et al., Sci. Reports of Meiji Seika Kaisha 31, 1992, S. 1-8 aus derselben Kultur wurden isoliert: PF 1022B, PF 1022C und PF 1022D: JP-Pat. 5170 749; PF 1022E: JP-Pat. 6 184 126; die unveröffentlichte PCT-Anmeldung PCT 965190; Totalsynthese der Cyclooktadepsipeptide: JP-Pat. 5 229 997; JP-Pat. 5 320 148; Makoto Ohyama et al., Biosci. Biotech. Biochem., 58 (6), 1994, S. 1193; Makoi Kobayashi et al., Annu. Rep. Sankyo Res. Lab. 46, 1994, S. 67; Stephen J. Nelson et al., J. Antibiotics 47 (11), 1994, S. 1322; J. Scherkenbeck et al. Tetrahedron 51 (31), 1995, S. 8459 (PF 1022A); B. H. Lee Tetrahedron Lett. 38 (5), 1997, S. 757; WO 93/ 19 053; EP-OS 634 408; WO 94/ 19 334; WO 95/ 07 272; EP-OS 626 375; EP-OS 626 376, EP-OS 664 297; EP 718 293; WO 96/11 945; WO 97/11 064 sowie WO 97/09 331 und WO 97/02 256].

Eine Vielzahl unterschiedlicher Schweflungsreagenzien sind in der Literatur beschrieben, wie beispielsweise Schwefelwasserstoff (H₂S), Schwefelwasserstoff/ Chlorwasserstoff (H₂S/HCl), Wasserstoffpersulfid/Chlorwasserstoff (H₂S₂/HCl), Di-(diethylaluminium)-sulfid [(Et₂Al)₂S], polymeres Ethylaluminiumsulfid [(EtAlS)ₙ], Siliziumdisulfid (SiS₂), Dibortrisulfid (B₂S₃), Phosphorpentachlorid/Dialuminiumtrisulfid/Natriumsulfat (PCl₅/Al₂S₃/Na₂SO₄), Natriumsulfid/Schwefelsäure (Na₂S/H₂SO₄), Diphosphorpentasulfid (P₂S₅), Diphosphorpentasulfid/Pyridin (P₂S₅/Py), Diethylthiocarbamoylchlorid, Diphosphorpentasulfid/Triethylamin (P₂S₅/NEt₃), Diphosphorpentasulfid/n-Butyllithium (P₂S₅/n-BuLi), Diphosphorpentasulfid/Natriumhydrogencarbonat (P₂S₅/NaHCO₃; "Scheeren's Reagens", Bildung von Na²⁺ [P₄S₁₀O]²⁻), Diphosphorpentasulfid/Methanol (P₂S₅/MeOH), SCN-CO- OEt, PSCl_{X} ^{·} (NMe₂)_{3-X} (X = 0-3), Bis(tricyclohexylzinn)sulfid/Bortrihalogenid [(C₆H₁₁)₃Sn]S₂+BX₃ (X=Cl, F), EP 0 280 867 (1988), Bis(1,5-cyclooktandiylboryl)sulfid [(9-BBN)₂S] als Schwefelungsreagens oder als Phosphorpentasulfid-Ersatz 2,4-Bis-(methylthio)-1,3,2,4-dithiadiphosphetan-2,4-disulfid "Davy-Reagens Methyl" (DR-Me), 2,4-Bis-(ethylthio)-1,3,2,4-dithiadiphosphetan-2,4-disulfid "Davy-Reagens Ethyl" (DR-Et), 2,4-Bis-(p-tolylthio)-1,3,2,4-dithiadiphosphetan-2,4-disulfid "Davy-Reagens p-Tolyl oder Heimgartner Reagens" (DR-T), 2,4-Bis-(4-phenoxyphenyl)-2,4-dithioxo-1,3,2,4-dithiadiphosphetan "Belleau's Reagens (BR)", 2,4-Bis-(4-phenylthiophenyl)-2,4-dithioxo-1,3,2,4-dithiadiphosphetan, 2,4-Bis-(4-methoxyphenyl)-2,4-dithioxo-1,3,2,4-dithiadiphosphetan "Lawesson's Reagens (LR)" (vgl. Davy-Reagens: H. Heimgartner et al., Helv. Chim. Acta 70, 1987, S. 1001; Belleau's Reagens: Tetrahedron 40, 1984, S. 2047; Tetrahedron 40, 1984, S. 2663; Tetrahedron Letters 24, 1983, S. 3815; I. Thomson et al., Org. Synth. 62, 1984, S. 158 sowie dort zitierte Literatur; D. Brillon Synthetic Commun. 20 (19), 1990, S. 3085 and dort zitierte Literatur; selektive Thionierung von Oligopeptiden: K. Clausen et al., J. Chem. Soc., Perkin Trans I 1984, 785; O. E. Jensen et al., Tetrahedron 41, 1985, S. 5595; Reviews über "Lawesson's Reagenz, (LR)": R. A. Cherkasov et al., Tetrahedron 41, 1985, S. 2567; M. P. Cava et al., Tetrahedron 41, 1985, S. 5061; Diborylsulfid: Liebigs Ann Chem. 1992, S. 1081 und dort zitierte Literatur; Metzner et al. in Sulfur Reagents in Organic Synthesis, B. Harcourt: London 1994, Academic Press, S. 44-45).

Alternativ sind auch Reaktionsfolgen, wie beispielsweise eine O-Alkylierung mit R₃O⁺BF₄⁻ (R: -Methyl, Ethyl) (H. Meerwein et al., Justus Liebigs Ann. Chem. 641, (1961) S. 1) und anschließende Umsetzung der Intermediate mit wasserfreiem NaSH (R. E. Eibeck, Inorg. Syn. 7, (1963) S. 128), die *in-situ* Bildung von Chloriminiumsalzen und nachfolgende Reaktion mit Tetrathiomolybdaten, insbesondere Benzyltriethylammoniumtetramolybdat [(Ph-CH₂-NEt₃)₂MoS₄] (Tetrahedron Lett. 36 (45), 1995, S. 8311) oder Hexamethyldisilathian (TMS₂S) (TMS: Trimethylsilyl; P. L. Fuchs et al., J. Org. Chem. 59, 1994, S. 348) möglich.

Zur Durchführung des erfindungsgemäßen Verfahrens 2 werden als Sulfidierungsreagenzien bevorzugt Phosphorreagenzien wie beispielsweise Diphosphorpentasulfid (P₂S₅), Diphosphorpentasulfid/Pyridin (P₂S₅/Py), Diphosphorpentasulfid/Triethylamin (P₂S₅/NEt₃), Diphosphorpentasulfid/Natriumhydrogencarbonat (P₂S₅/ NaHCO₃ "Scheeren's Reagens") oder besonders bevorzugt das racemisierungsfreie 2,4-Bis-(4-methoxyphenyl)-2,4-dithioxo-1,3,2,4-dithiadiphosphetan (LR: Lawesson's Reagens) (K. Clausen, M. Thorsen, S.-O. Lawesson Tetrahedron 37, 1981, S. 3635) 2,4-Bis-(4-phenoxyphenyl)-2,4-dithioxo-1,3,2,4-dithiadiphosphetan "Belleau's Reagens (BR)" bzw. 2,4-Bis-(4-phenylthiophenyl)-2,4-dithioxo-1,3,2,4-dithiadiphosphe-tan, eingesetzt.

Im allgemeinen ist es vorteilhaft, das erfindungsgemäße Verfahren in Gegenwart von Verdünnungsmitteln durchzuführen. Verdünnungsmittel werden vorteilhaft in einer solchen Menge eingesetzt, daß das Reaktionsgemisch während des ganzen Verfahrens gut rührbar bleibt. Als Verdünnungsmittel zur Durchführung des erfindungsgemäßen Verfahrens kommen alle inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids: Amine wie Trimethyl-, Triethyl-, Tripropyl-, Tributylamin, N-Methyl-morpholin, Pyridin und Tetramethylendiamin, Nitrokohlenwasserstoffe wie Niromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwas-serstoffe wie Pentan, Hexan, Heptan, Oktan, Nonan und technische Kohlenwasserstoffe, beispielsweise sogenannte White Spirits mit Komponenten mit Siedepunkten im Bereich beispielsweise von 40°C bis 250°C, Cymol, Benzinfraktionen innerhalb eines Siedepunktintervalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphortriamid, Formamid, N-Methyl-formamid, N,N-Dimethylformamid, N,N-Dipropylformamid, N,N-Dibutylformamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-1,4-Diformylpiperazin; Ketone wie Aceton, Acetophenon, Methyl-ethylketon, Methylbutylketon.

Selbstverständlich kann man das erfindungsgemäße Verfahren auch in Gemischen der genannten Lösungs- und Verdünnungsmittel durchführen.

Die zu verwendenden Verdünnungsmittel sind vom jeweils eingesetzten Schwefelungsreagens abhängig.

Bevorzugte Verdünnungsmittel zur Thionierung sind jedoch aromatische Kohlenwasserstoffe wie Benzol, Toluol, Chlorbenzol, Brombenzol, Nitrobenzol oder Xylol, Ether wie Ethylpropylether, Methyl-tert-butylether, Anisol, Phenetol, Cyclohexylmethylether, Tetrahydrofuran oder Dioxan.

Die Thionierung nach Verfahren 2 wird durchgeführt, indem man die Depsipeptide der allgemeinen Formel ( II ) in Gegenwart eines geeigneten Schweflungsreagens, beispielsweise [2,4-Bis-(4-methoxyphenyl)-2,4-dithioxo-1,3,2,4-dithiadiphosphetan (LR. Lawesson's Reagens), 2,4-Bis-(4-phenoxyphenyl)-2,4-dithioxo-1,3,2,4-dithiadiphosphetan (BR: Belleau's Reagens) bzw. 2,4-Bis-(4-phenylthiophenyl)-2,4-dithioxo-1,3,2,4-dithiadiphosphetan, in einem der angegebenen Verdünnungsmittel umsetzt.

Die Reaktionsdauer beträgt 10 Minuten bis 72 Stunden. Die Umsetzung erfolgt bei Temperaturen zwischen -10 °C und +200 °C, bevorzugt zwischen 0 °C und +150 °C, besonders bevorzugt bei Temperaturen zwischen +10 °C und + 130 °C oder bei Siedetemperatur eines geeigneten Verdünnungsmittels. Es kann grundsätzlich unter Normaldruck gearbeitet werden. Vorzugsweise arbeitet man bei Normaldruck oder bei Drucken bis zu 15 bar. und gegebenenfalls unter Schutzgasatmosphäre (Stickstoff oder Helium).

Zur Durchführung des erfindungsgemäßen Verfahrens 2 setzt man pro vorhandener Amidcarbonylgruppe in Verbindungen der allgemeinen Formeln ( II ) im allgemeinen 0,5 bis 3,5 Mol, vorzugsweise 1,0 bis 2,5 Mol an Schweflungsreagens ein.

Nach vollendeter Thionierung wird der gesamte Reaktionsansatz abgekühlt, vom ausfallenden Thionierungsmittel abgetrennt und gegebenenfalls gewaschen. Die anfallenden Produkte lassen sich in üblicher Weise durch Umkristallisieren, Vakuumdestillation oder Säulenchromatographie reinigen (vgl. auch die Herstellungsbeispiele).

Mit dem Verfahren sind, aus den einzelnen Bausteinen mit sowohl (S)- als auch (R)-Konfiguration (bzw. L- and D-Konfiguration), Thiodepsipeptide unter Beibehaltung der ursprünglichen Konfiguration der Ausgangsstoffe erhältlich.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acanthocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp , Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbander, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emutsionen und Suspensionen zur oralen oder dermalen Anwendung sowie zur Injektion; halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter ebentuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂₋C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.
Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz.

Als weitere Hilfsstoffe seien genannt: Viskositätserhöhende und die Emulsion stabilisierende Stoffe wie Carboxymethylcellulose, Methylcellulose und andere Celluloseund Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden, enthalten den Wirkstoff in Konzentrationen von 0,5 - 90 Gew.-%, bevorzugt von 5 - 50 Gew.-%.

### Beispiel A

### In vivo Nematodentest

### Heterakis spumosa / Maus

Mäuse werden experimentell mit Nematoden der Art *Heterakis spumosa* infiziert. Zur Infektion wird den Mäusen oral 90 embryonierte *Heterakis spumosa* Eier appliziert.

Nach Ablauf der Präpatenzzeit werden die suspendierten Wirkstoffe oral und/oder intraperitoneal am 46. Tag nach der Infektion appliziert.

### Bestimmung der Wirksamkeit:

Die Sektion der Mäuse erfolgt am 54. Tag nach der Infektion. Die Auszählung der adulten Parasiten im Colon und Caecum wird mikroskopisch durchgeführt. Der Behandlungserfolg in der Dosisgruppe wird ins Verhältnis zur unbehandelten Kontrollgruppe gesetzt.

Geprüfte Wirkstoffe und wirksame Dosierungen (Dosis effectiva) sind aus der nachfolgenden Tabelle ersichtlich.

| Wirkstoff Beispiel-Nr. | Dosis effectiva in [mg/kg] | Reduktionsrate in [%] |
|---|---|---|
| PF 1022A | 50 | 0 |
| 1 | 50 | 100 |

### Beispiel B

### In vivo Nematodentest

### Haemonchus contortus / Schaf

Experimentell mit *Haemonchus contortus* infizierte Schafe wurden nach Ablauf der Präpatenzzeit des Parasiten behandelt. die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel-Nr. | Dosis effectiva in [mg/kg] |
|---|---|
| Cyclo(-MeLeu-D-Lac-MeLeu-D-PhLac-)₂ PF 1022A | 0.25 |
| Cyclo(-MeLeu-D-Lac-MeLeu-D-4-MorPhLac-)₂ | 0.05 |
| 1 | 0.10 |
| 3 | 0.025 |
| 6 | 0.01 |
| 7 | 0.01 |
| 8 | 0.01 |
| 10 | 0.01 |
| 12 | 0.01 |
| D-4-MorPhLac: D-4-Morpholinophenyllactat | |

### Herstellungsbeispiele

### Beispiel 1

### Cyclo(-N-methyl-L-leucinyl-D-thiolactyl-N-methyl-L-leucinyl-D-phenylthiolactyl-N-methyl-L-leucinyl-D-thiolactyl-N-methyl-L-leucinyl-D-phenylthiolactyl-)

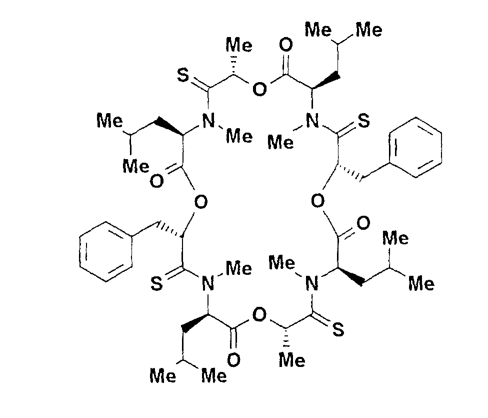

1,0 g (1,05 mMol) Cyclo(-N-methyl-L-leucinyl-D-lactyl-N-methyl-L-leucinyl-Dphenyllactyl-N-methyl-L-leucinyl-D-lactyl-N-methyl-L-leucinyl-D-phenyllactyl-) PF 1022A *(vgl. EP-OS 382 173, US-Pat*. *5116 815)* wurden in 20 ml Toluol mit 1,4 g (3,5 mMol) 2,4-Bis-(4-methoxy-phenyl)-2,4-dithioxo-1,3,2,4-dithiadiphosphetan ("Lawesson's Reagens") versetzt und 3,5 Stunden unter Rückflußtemperatur gerührt. Anschließend wird der gesamte Reaktionsansatz auf 0 °C abgekühlt, filtriert und das erhaltene Filtrat im Vakuum eingeengt. Das zurückbleibende Rohprodukt wird über eine Kieselgelsäule (Kieselgel 60 - Merck, Korngröße: 0,04 bis 0,063 mm) zunächst mit dem Fließmittel Methylenchlorid und anschließend mit dem Fließmittel Cyclohexan : Aceton ( 3 : 1 ) chromatographiert. Man erhält 0,46 g (43,6 % der Theorie) Cyclo(-N-methyl-L-leucinyl-D-thiolactyl-N-methyl-L-leucinyl-D-phenylthiolactyl-N-methyl-L-leucinyl-D-thiolactyl-N-methyl-L-leucinyl-D-phenylthiolactyl-).
¹H-NMR (CDCl₃, δ): 2,99, 3,06, 3,26, 3,42 (4 x -N-Me); 4,86, 6,42, 6,61 (4 x -N-CH₂-);
5,31, 5,55, 5,81, 5,89 (4 x -O-CH₂-); 7,26 (Phenyl-H) ppm.
LC-MS (sauer) m/z (%): 1013 (M⁺, 100); 310 (21); 274 (30); 198 (42).
*C*_{*52*}*H*_{*76*}*N*_{*4*}*O*_{*8*}*S*_{*4*} *(1013*.*4)*

### Beispiel 2

### Cyclo(-N-methyl-L-leucinyl-D-thiolactyl-N-methyl-L-leucinyl-D-4-nitro-phenylthiolactyl-N-methyl-L-leucinyl-D-thiolacyl-N-methyl-L-leucinyl-D-4-nitro-phenylthiolactyl-)

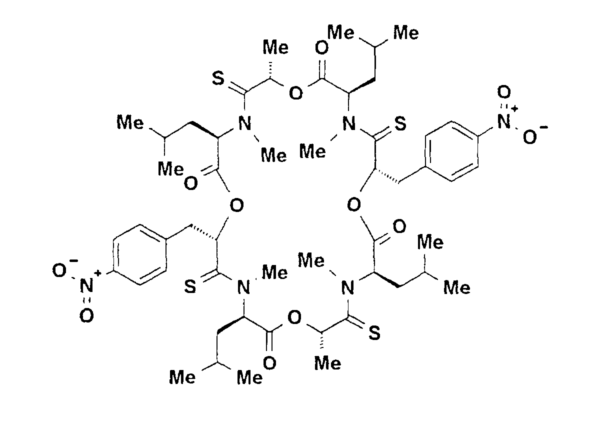

Die Thionierung erfolgt analog der Reaktionsvorschrift des Beispiels 1 unter Verwendung von:
- 0,50 g (0,48 mMol): Cyclo(-N-methyl-L-leucinyl-D-lactyl-N-methyl-L-leucinyl-D-4-nitro-phenyllactyl-N-methyl-L-leucinyl-D-lactyl-N-methyl-L-leucinyl-D-4-nitro-phenyllactyl-) *(vgl. WO 93*/*19 053*, *EP-OS 634 408)*
- 0,65 g (1,59 mMol): 2,4-Bis-(4-methoxy-phenyl)-2,4-dithioxo-1,3,2,4-dithiadiphosphetan ("Lawesson's Reagens")
- 10 ml: absolutes Toluol

Das zurückbleibende Rohprodukt wird über eine Kieselgelsäule (Kieselgel 60 - Merck, Korngröße: 0,04 bis 0,063 mm) zunächst mit dem Fließmittel Methylenchlorid und anschließend mit dem Fließmittel Cyclohexan : Aceton ( 3 : 1 ) chromatographiert Man erhält 0,34 g (63,8 % der Theorie) Cyclo(-N-methyl-L-leucinyl-Dthiolac-tyl-N-methyl-L-leucinyl-D-4-nitro-phenylthiolactyl-N-methyl-L-leucinyl-Dthiolactyl-N-methyl-L-leucinyl-D-4-nitro-phenylthiolactyl-).
¹H-NMR (CDCl₃, δ): 3,04, 3,09, 3,25, 3,50 (4 x -N-Me); 4,87, 6,38, 6,56, 6,63 (4 x - N-CH₂-); 5,31, 5,52, 5,81, 5,91 (4 x -O-CH₂-); 8,17; 7,46 (Aryl-H) ppm.
LC-MS (sauer) m/z (%): 1103 (M+H, 100); 392 (38); 177 (40); 136 (30).
*C*_{*52*}*H*_{*74*}*N*_{*6*}*O*_{*12*}*S*_{*4*} *(1103,4)*

### Beispiel 3

### Cyclo(-N-methyl-L-leucinyl-D-thiolactyl-N-methyl-L-leucinyl-D-4-N-morpholinophenylthiolactyl-N-methyl-L-leucinyl-D-thiolactyl-N-methyl-L-leucinyl-D-4-N-morpholino-phenylthiolactyl-)

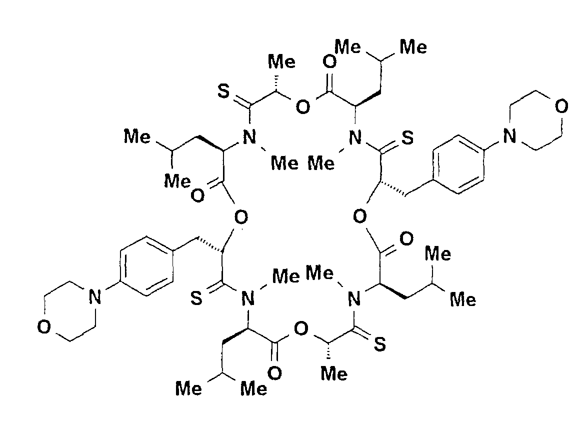

Die Thionierung erfolgt analog der Reaktionsvorschrift des Beispiels 1 unter Verwendung von:
- 0,50 g (0,44 mMol): Cyclo(-N-methyl-L-leucinyl-D-lactyl-N-methyl-L-leucinyl-D-4-N-morpholino-phenyllactyl-N-methyl-L-leucinyl-Dlactyl-N-methyl-L-leucinyl-D-4-N-morpholino-phenyllactyl-) *(vgl. WO 93*/*19 053, EP-OS 634 408)*
- 0,60 g (1,48 mMol): 2,4-Bis-(4-methoxy-phenyl)-2,4-dithioxo-1,3,2,4-dithiadiphos-phetan ("Lawesson's Reagens")
- 10 ml: absolutes Toluol

Das zurückbleibende Rohprodukt wird über eine Kieselgelsäule (Kieselgel 60 - Merck, Korngröße: 0,04 bis 0,063 mm) zunächst mit dem Fließmittel Methylenchlorid und anschließend mit dem Fließmittel Cyclohexan : Aceton ( 3 : 1 ) chromatographiert. Man erhält 0,37 g (70,0 % der Theorie) Cyclo(-N-methyl-L-leucinyl-D-thiolactyl-N-methyl-L-leucinyl-D-4-N-morpholino-phenylthiolactyl-N-methyl-L-leucinyl-D-thiolactyl-N-methyl-L-leucinyl-D-4-N-morpholino-phenylthiolactyl-).
¹H-NMR (CDCl₃, δ): 3,01, 3,08, 3,26, 3,40 (4 x -N-Me); 3,12, 3,85 (2 x Mor); 4,85, 6,42, 6,62 (4 x -N-CH₂-); 5,30, 5,55, 5,78, 5,88 (4 x -O-CH₂-); 6,81, 7,26 (Aryl-H) ppm.
LC-MS (sauer) m/z (%): 1184 (M+H, 100); 986 (13); 593 (32); 392 (73); 177 (78).
*C*_{*60*}*H*_{*90*}*N*_{*6*}*O*_{*10*}*S*_{*4*} *(1183,6)*

### Beispiel 4

### Cyclo(-N-methyl-L-leucinyl-D-thiolactyl-N-methyl-L-leucinyl-D-4-(fur-2-yl-methoxy)-phenylthiolactyl-N-methyl-L-leucinyl-D-thiolactyl-N-methyl-L-leucinyl-Dphenylthiolactyl-)

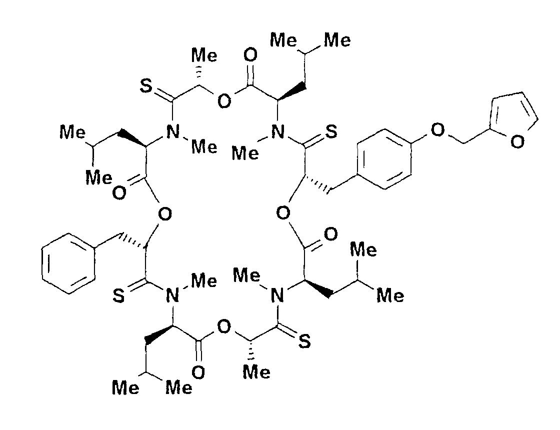

Die Thionierung erfolgt analog der Reaktionsvorschrift des Beispiels 1 unter Verwendung von:
- 0,50 g (0,48 mMol): Cyclo(-N-methyl-L-leucinyl-D-lactyl-N-methyl-L-leucinyl-D-4-(fur-2-yl-methoxy)-phenyllactyl-N-methyl-L-leucinyl-Dlactyl-N-methyl-L-leucinyl-D-phenyllactyl-) *(vgl. WO 97*/*11 064))*
- 0,64 g (1,58 mMol): 2,4-Bis-(4-methoxy-phenyl)-2,4-dithioxo-1,3,2,4-dithiadiphosphetan ("Lawesson's Reagens")
- 10 ml: absolutes Toluol

Das zurückbleibende Rohprodukt wird über eine Kieselgelsäule (Kieselgel 60 - Merck, Korngröße: 0,04 bis 0,063 mm) zunächst mit dem Fließmittel Methylenchlorid und anschließend mit dem Fließmittel Cyclohexan : Aceton ( 3 : 1 ) chromatographiert Man erhält 0,14 g (26,6 % der Theorie) Cyclo(-N-methyl-L-leucinyl-Dthiolactyl-N-methyl-L-leucinyl-D-4-(fur-2-yl-methoxy)-phenylthiolactyl-N-methyl-Lleucinyl-D-thiolactyl-N-methyl-L-leucinyl-D-phenylthiolactyl-).
F.: 120-125 °C
¹H-NMR (CDCl₃, δ): 3,15, 3,21, 3,42, 3,57 (4 x -N-Me); 5,03, 6,55, 6,78 (4 x -N-CH₂-);
5,45, 5,69, 5,89-6,08 (4 x -O-CH₂-); 7,05-7,60 (Aryl-H, Furyl-H) ppm.
LC-MS (loop) m/z (%): 1109 (M⁺, 2); 1108 (3); 370 (15); 274 (32).
*C*_{*57*}*H*_{*80*}*N*_{*4*}*O*_{*10*}*S*_{*4*} *(1109,5)*

### Beispiel 5

### Cyclo(-N-methyl-L-leucinyl-D-thiolactyl-N-methyl-L-leucinyl-D-4-(fur-2-yl-methoxy)-phenylthiolactyl-N-methyl-L-leucinyl-D-thiolactyl-N-methyl-L-leucinyl-D-4-(fur-2-yl-methoxy)-phenylthiolactyl-)

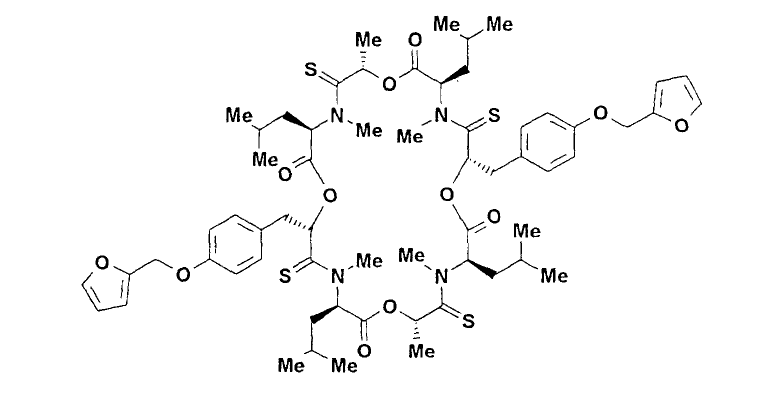

Die Thionierung erfolgt analog der Reaktionsvorschrift des Beispiels 1 unter Verwendung von:
- 0,50 g (0,44 mMol): Cyclo(-N-methyl-L-leucinyl-D-lactyl-N-methyl-L-leucinyl-D-4-(fur-2-yl-methoxy)-phenyllactyl-N-methyl-L-leucinyl-Dlactyl-N-methyl-L-leucinyl-D-4-(fur-2-yl-methoxy)-phenyllactyl-) *(vgl. WO 97*/*11 064)*
- 0,58 g (1,44 mMol): 2,4-Bis-(4-methoxy-phenyl)-2,4-dithioxo-1,3,2,4-dithiadiphosphetan ("Lawesson's Reagens")
- 10 ml: absolutes Toluol

Das zurückbleibende Rohprodukt wird über eine Kieselgelsäule (Kieselgel 60 - Merck, Korngröße: 0,04 bis 0,063 mm) zunächst mit dem Fließmittel Methylenchlorid und anschließend mit dem Fließmittel Cyclohexan : Aceton ( 3 : 1 ) chromatographiert. Man erhält 0,12 g (22,7 % der Theorie) Cyclo(-N-methyl-L-leucinyl-D-thiolactyl-N-methyl-L-leucinyl-D-4-(fur-2-yl-methoxy)-phenylthiolactyl-N-methyl-L-leucin-yl-D-thiolactyl-N-methyl-L-leucinyl-D-4-(fur-2-yl-methoxy)-phenylthiolactyl-).
F.: 107-110 °C
¹H-NMR (CDCl₃, δ): 3,01, 3,07, 3,26, 3,41 (4 x -N-Me); 4,96 (2 x -O-CH₂-); 4,86, 6,42, 6,62 (4 x -N-CH₂-); 5,32, 5,56, 5,78, 5,85 (4 x -O-CH₂-); 6,91-7,44 (Aryl-H, Furyl-H) ppm.
LC-MS (sauer) m/z (%): 1205 (M⁺, 3); 1204 (5); 370 (41); 198 (100).
*C*_{*62*}*H*_{*84*}*N*_{*4*}*O*_{*12*}*S*_{*4*} *(1205,6)*

### Beispiel 6

### Cyclo(-N-methyl-L-leucinyl-D-thiolactyl-N-methyl-L-leucinyl-D-4-(pyrid-2-ylmethoxy)-phenylthiolactyl-N-methyl-L-leucinyl-D-thiolactyl-N-methyl-L-leu-cinyl-D-4-(pyrid-2-yl-methoxy)-phenylthiolactyl-)

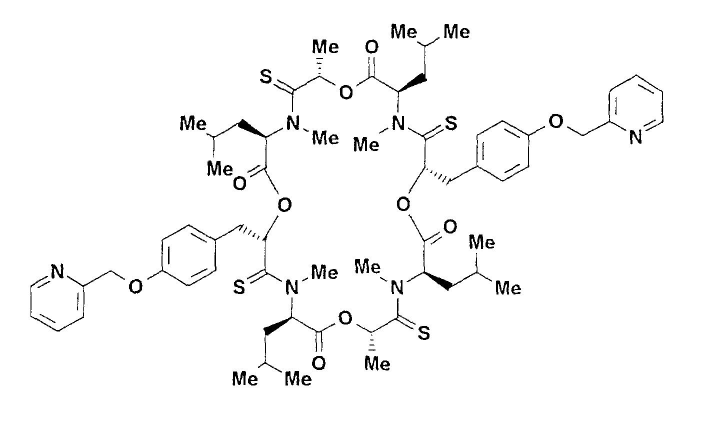

Die Thionierung erfolgt analog der Reaktionsvorschrift des Beispiels 1 unter Verwendung von:
- 0,50 g (0,43 mMol): Cyclo(-N-methyl-L-leucinyl-D-lactyl-N-methyl-L-leucinyl-D-4-(pyrid-2-yl-methoxy)-phenyllactyl-N-methyl-L-leucinyl-D-lactyl-N-methyl-L-leucinyl-D-4-(pyrid-2-yl-methoxy)-phenyllactyl-) *(vgl. WO 97*/*11 064)*
- 0,57 g (1,41 mMol): 2,4-Bis-(4-methoxy-phenyl)-2,4-dithioxo-1,3,2,4-dithiadiphosphetan ("Lawesson's Reagens")
- 10 ml: absolutes Toluol

Das zurückbleibende Rohprodukt wird über eine Kieselgelsäule (Kieselgel 60 - Merck, Korngröße: 0,04 bis 0,063 mm) zunächst mit dem Fließmittel Methylenchlorid und anschließend mit dem Fließmittel Cyclohexan : Aceton ( 3 : 1 ) chromatographiert Man erhält 0,16 g (30,9 % der Theorie) Cyclo(-N-methyl-L-leucinyl-D-thiolactyl-N-methyl-L-leucinyl-D-4-(pyrid-2-yl-methoxy)-phenylthiolactyl-N-methyl-Lleucinyl-D-thiolactyl-N-methyl-L-leucinyl-D-4-(pyrid-2-yl-methoxy)-phenylthiolactyl-).
¹³C-NMR (CDCl_{3,} δ): 34,8, 35,2, 37,1, 39,2 (N-Me); 71,8, 73,3, 75,4 (-CH-O-); 40,1, 40,6 (-CH₂-); 62,3, 62,5, 62,6 (-CH-N-); 121,4, 122,8, 136,9, 149,4 (=CH-, Py); 70,7 (-CH₂-O-); 115,0, 130,9 (=CH-, Phenyl); 203,2, 203,4, 204,8, 205,7 (N-C=S); 168,8, 169,5, 170,0 (-O-C=O) ppm.
¹H-NMR (CDCl_{3,} δ): 3,01, 3,19, 3,26, 3,41 (4 x -N-Me); 5,17 (2 x -O-CH₂-); 4,86, 6,41, 6,64, 6,66 (4 x -N-CH₂-); 5,31, 5,75, 5,57, 5,85 (4 x -O-CH₂-); 6,91, 7,16 (Phenyl-H), 7,24, 7,51, 7,72, 8,59 (Pyridyl-H) ppm.
LC-MS (loop) m/z (%): 1228 (M+H, 18); 383 (58); 224 (100).
*C*_{*64*}*H*_{*86*}*N*_{*6*}*O*_{*14*}*S*_{*4*} *(1227,6)*

### Beispiel 7

### Cyclo(-N-methyl-L-leucinyl-D-thiolactyl-N-methyl-L-leucinyl-D-phenyllactyl-N-methyl-L-leucirzyl-D-lactyl-N-methyl-L-leucinyl-D-phenyllactyl-)

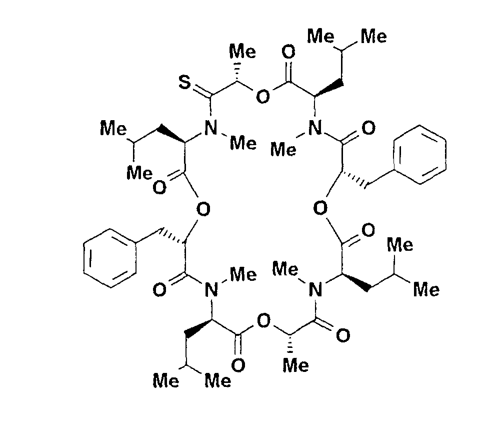

1,0 g (1,05 mMol) Cyclo(-N-methyl-L-leucinyl-D-lactyl-N-methyl-L-leucinyl-D-phenyllactyl-N-methyl-L-leucinyl-D-lactyl-N-methyl-L-leucinyl-D-phenyllactyl-) PF 1022A *(vgl. EP-OS 382173, US-Pat. 5116815)* wurden in 15 ml Tetrahydrofuran bei 0°C mit 0,26 g (0,05 mMol) 2,4-Bis-(4-phenoxy-phenyl)-2,4-dithioxo-1,3,2,4-dithiadiphosphetan ("Belleau's Reagens") versetzt und 18 Stunden bei Raumtemperatur gerührt. Anschließend wird der gesamte Reaktionsansatz im Vakuum eingeengt. Das zurückbleibende Rohprodukt wird zweimal über eine Kieselgelsäule (Kieselgel 60 - Merck, Korngröße: 0,04 bis 0,063 mm) mit dem Fließmittel Cyclohexan : Aceton ( 3 : 1 ) chromatographiert. Man erhält 0,12 g (11,8 % der Theorie) Cyclo(-N-methyl-L-leucinyl-D-thiolactyl-N-methyl-L-leucinyl-D-phenyllactyl-N-methyl-L-leucinyl-D-lac-tyl-N-methyl-L-leucinyl-D-phenyllactyl-).
LC-MS (sauer) m/z (%): 965 (M⁺, 100); 200 (45).
*C*_{*52*}*H*_{*76*}*N*_{*4*}*O*_{*11*}*S (965,2)*

### Beispiel 8

### Cyclo(-N-methyl-L-leucinyl-D-thiolactyl-N-methyl-L-leucinyl-D-4-N-morpholinophenyllactyl-N-methyl-L-leucinyl-D-lactyl-N-methyl-L-leucinyl-D-4-N-morpholinophenyllactyl-)

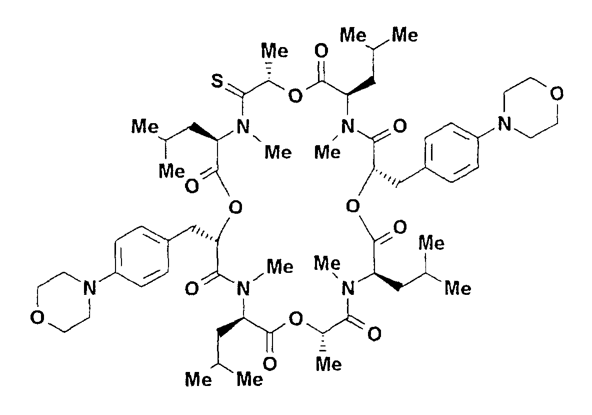

Die Thionierung erfolgt analog der Reaktionsvorschrift des Beispiels 7 unter Verwendung von:
- 0,41 g (0,37 mMol): Cyclo(-N-methyl-L-leucinyl-D-lactyl-N-methyl-L-leucinyl-D-4-N-morpholino-phenyllactyl-N-methyl-L-leucinyl-Dlactyl-N-methyl-L-leucinyl-D-4-N-morpholino-phenyllactyl-) *(vgl*. *WO 93*/*19 053, EP-OS 634 408)*
- 0,36 g (0,73 mMol): 2,4-Bis-(4-phenoxy-phenyl)-2,4-dithioxo-1,3,2,4-dithiadiphosphetan ("Belleau's Reagens")
- 10 ml: absolutes Toluol

Das zurückbleibende Rohprodukt wird über eine Kieselgelsäule (Kieselgel 60 - Merck, Korngröße: 0,04 bis 0,063 mm) mit dem Fließmittel Cyclohexan Aceton (3 : 1) chromatographiert. Man erhält 0,70 g (16,8 % der Theorie) Cyclo(-N-methyl-L-leucinyl-D-thiolactyl-N-methyl-L-leucinyl-D-4-N-morpholino-phenyllactyl-N-methyl-L-leucinyl-D-lactyl-N-methyl-L-leucinyl-D-4-N-morpholino-phenyllactyl-).
LC-MS (sauer) m/z (%): 1135 (M⁺, 56); 361 (100).
*C*_{*60*}*H*_{*90*}*N*_{*6*}*O*_{*13*}*S (1135,4)*
Rₜ (HPLC): 16.53 min.

### Beispiel 9

### Cyclo(-N-methyl-L-leucinyl-D-thiolactyl-N-methyl-L-leucinyl-D-4-nitro-phenyllactyl-N-methyl-L-leucinyl-D-lactyl-N-methyl-L-leucinyl-D-4-nitro-phenyllactyl-)

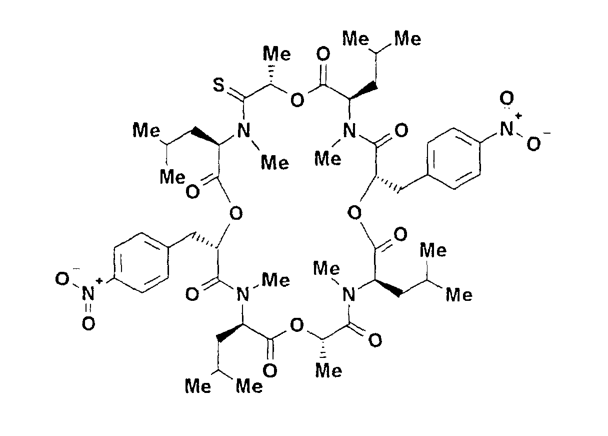

Die Thionierung erfolgt analog der Reaktionsvorschrift des Beispiels 7 unter Verwendung von:
- 0,50 g (0,48 mMol): Cyclo(-N-methyl-L-leucinyl-D-lactyl-N-methyl-L-leucinyl-D-4-nitro-phenyllactyl-N-methyl-L-leucinyl-D-lactyl-N-methyl-L-leucinyl-D-4-nitro-phenyllactyl-) *(vgl*. *WO 93*/*19 053*, *EP-OS 634 408)*
- 0,24 g (0,48 mMol): 2,4-Bis-(4-phenoxy-phenyl)-2,4-dithioxo-1,3,2,4-dithiadiphosphetan ("Belleau's Reagens")
- 10 ml: absolutes Tetrahydrofuran

Man rührt das Reaktionsgemisch 24 Stunden bei 50 °C und engt im Vakuum ein. Das zurückbleibende Rohprodukt wird über eine Kieselgelsäule (Kieselgel 60 - Merck, Korngröße: 0,04 bis 0,063 mm) mit dem Fließmittel Cyclohexan : Aceton ( 4 : 1) chromatographiert. Anschließend wird das Produkt nochmals mittels präparativer HPLC (Gradient: Wasser/Acetonitril) gereinigt. Man erhält 16 mg (3,1 % der Theorie) Cyclo(-N-methyl-L-leucinyl-D-thiolactyl-N-methyl-L-leucinyl-D-4-nitro-phenyllactyl-N-methyl-L-leucinyl-D-lactyl-N-methyl-L-leucinyl-D-4-nitro-phenyllactyl-).
LC-MS (sauer) m/z (%): 1056 (M+H, 38).
*C*_{*52*}*H*_{*74*}*N*_{*6*}*O*_{*15*}*S (1055,3)*
Rₜ(HPLC): 17,38 min.

### Beispiel 10

### Cyclo(-N-methyl-L-leucinyl-D-thiolactyl-N-methyl-L-leucinyl-D-4-(pyrid-2-ylmethoxy)-phenyllactyl-N-methyl-L-leucinyl-D-lactyl-N-methyl-L-leucinyl-D-4-(pyrid-2-yl-methoxy)-phenyllactyl-)

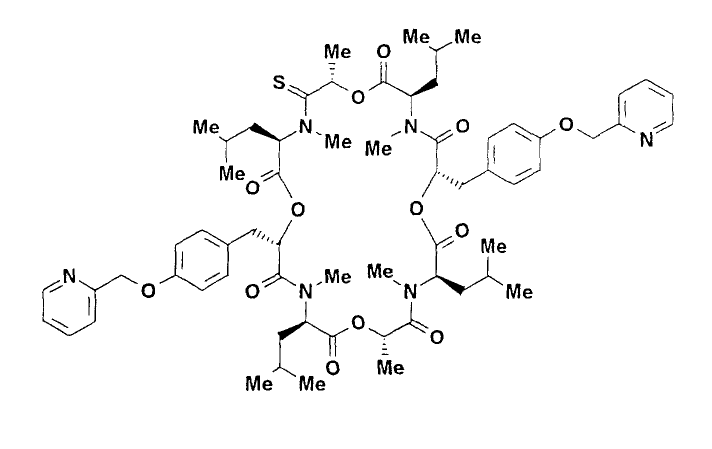

Die Thionierung erfolgt analog der Reaktionsvorschrift des Beispiels 9 unter Verwendung von:
- 0,41 g (0,35 mMol): Cyclo(-N-methyl-L-leucinyl-D-lactyl-N-methyl-L-leucinyl-D-4-(pyrid-2-yl-methoxy)-phenyllactyl-N-methyl-L-leucinyl-Dlactyl-N-methyl-L-leucinyl-D-4-(pyrid-2-yl-methoxy)-phenyllactyl-) *(vgl. WO 97*/*11 064)*
- 0,52 g (1,05 mMol): 2,4-Bis-(4-phenoxy-phenyl)-2,4-dithioxo-1,3,2,4-dithiadiphosphetan ("Belleau's Reagens")
- 10 ml: absolutes Tetrahydrofuran

Das zurückbleibende Rohprodukt wird über eine Kieselgelsäule (Kieselgel 60 - Merck, Korngröße: 0,04 bis 0,063 mm) zunächst mit dem Fließmittel Methylenchlorid und anschließend mit dem Fließmittel Cyclohexan : Aceton ( 4 : 1 ) chromatographiert. Man erhält 53,9 mg (13,0 % der Theorie) Cyclo(-N-methyl-L-leucinyl-Dthiolactyl-N-methyl-L-leucinyl-D-4-(pyrid-2-yl-methoxy)-phenyllactyl-N-methyl-Lleu-cinyl-D-lactyl-N-methyl-L-leuciny(-D-4-(pyrid-2-yl-methoxy)-phenyllactyl-).
¹³C-NMR (CDCl₃, δ): 204,9; 205,8 ppm (-NMe-C=S)/ 2 Konformationsisomere.
LC-MS (loop) m/z (%): 1179 (M⁺, 100).
*C*_{*64*}*H*_{*86*}*N*_{*6*}*O*_{*13*}*S (1179,5)*

### Beispiel 11

### Cyclo(-N-methyl-L-leucinyl-D-thiolactyl-N-methyl-L-leucinyl-D-4-(fur-2-yl-methoxy)-phenyllactyl-N-methyl-L-leucinyl-D-lactyl-N-methyl-L-leucinyl-D-4-(fur-2-ylmethoxy)-phenyllactyl-)

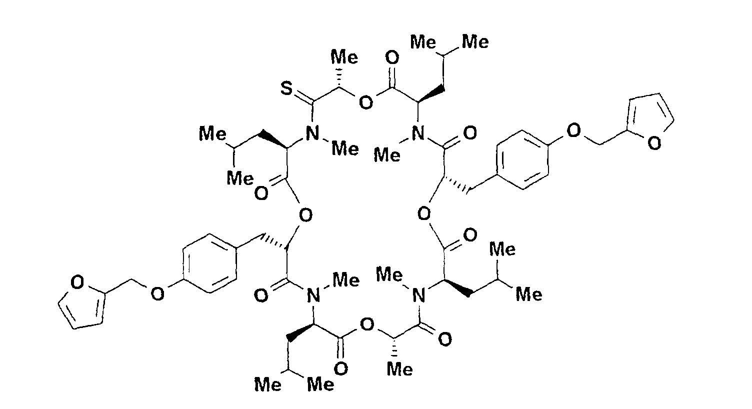

Die Thionierung erfolgt analog der Reaktionsvorschrift des Beispiels 9 unter Verwendung von:
- 0,47 g (0,41 mMol): Cyclo(-N-methyl-L-leucinyl-D-lactyl-N-methyl-L-leucinyl-D-4-(fur-2-yl-methoxy)-phenyllactyl-N-methyl-L-leucinyl-Dlactyl-N-methyl-L-leucinyl-D-4-(fur-2-yl-methoxy)-phenyllactyl-) *(vgl. WO 97*/*11 064)*
- 0,20 g (0,41 mMol): 2,4-Bis-(4-phenoxy-phenyl)-2,4-dithioxo-1,3,2,4-dithiadiphosphetan ("Belleau's Reagens")
- 10 ml: absolutes Tetrahydrofuran

Das zurückbleibende Rohprodukt wird über eine Kieselgelsäule (Kieselgel 60 - Merck, Korngröße: 0,04 bis 0,063 mm) zunächst mit dem Fließmittel Methylenchlorid und anschließend mit dem Fließmittel Cyclohexan : Aceton ( 4 : 1 ) chromatographiert. Man erhält 130 mg (27,3 % der Theorie) Cyclo(-N-methyl-L-leucinyl-D-thiolactyl-N-methyl-L-leucinyl-D-4-(fur-2-yl-methoxy)-phenyllactyl-N-methyl-L-leucinyl-D-lactyl-N-methyl-L-leucinyl-D-4-(fur-2-yl-methoxy)-phenyllactyl-).
¹³C-NMR (CDCl₃, δ): 204,9; 205,8 ppm (-NMe-C=S) / 2 Konformationsisomere.
LC-MS (sauer) m/z (%): 1158 (M+H, 100).
*C*_{*62*}*H*_{*84*}*N*_{*4*}*O*_{*15*}*S (1157,4)*
Rₜ (HPLC): 17,85 min.

### Beispiel 12

### Cyclo(-N-methyl-L-leucinyl-D-thiolactyl-N-methyl-L-leucinyl-D-4-(fur-2-yl-methoxy)-phenyllactyl-N-methyl-L-leucinyl-D-lactyl-N-methyl-L-leucinyl-D-phenyllactyl-)

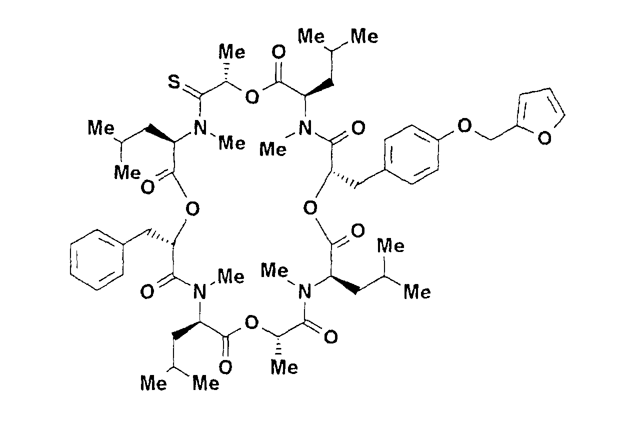

Die Thionierung erfolgt analog der Reaktionsvorschrift des Beispiels 9 unter Verwendung von:
- 0,40 g (0,38 mMol): Cyclo(-N-methyl-L-leucinyl-D-lactyl-N-methyl-L-leucinyl-D-4-(fur-2-yl-methoxy)-phenyllactyl-N-methyl-L-leucinyl-Dlactyl-N-methyl-L-leucinyl-D-phenyllactyl-) *(vgl*. *WO 97*/*11 064)*
- 0,20 g (0,38 mMol): 2,4-Bis-(4-phenoxy-phenyl)-2,4-dithioxo-1,3,2,4-dithiadiphosphetan ("Belleau's Reagens")
- 10 ml: absolutes Tetrahydrofuran

Das zurückbleibende Rohprodukt wird über eine Kieselgelsäule (Kieselgel 60 - Merck, Korngröße: 0,04 bis 0,063 mm) zunächst mit dem Fließmittel Methylenchlorid und anschließend mit dem Fließmittel Cyclohexan : Aceton ( 4 : 1 ) chromatographiert Man erhält 46,1 mg (11,4 % der Theorie) Cyclo(-N-methyl-L-leucinyl-Dthiolactyl-N-methyl-L-leucinyl-D-4-(fur-2-yl-methoxy)-phenyllactyl-N-methyl-Lleuci-nyl-D-lactyl-N-methyl-L-leucinyl-D-phenyllactyl-).
LC-MS (loop) m/z (%): 1061 (M⁺, 100).
*C*_{*57*}*H*_{*80*}*N*_{*4*}*O*_{*13*}*S (1061,3)*
Rₜ (HPLC): 17,55 min.

## Patentansprüche

1. Verbindungen der allgemeinen Formel (I) und deren Salze 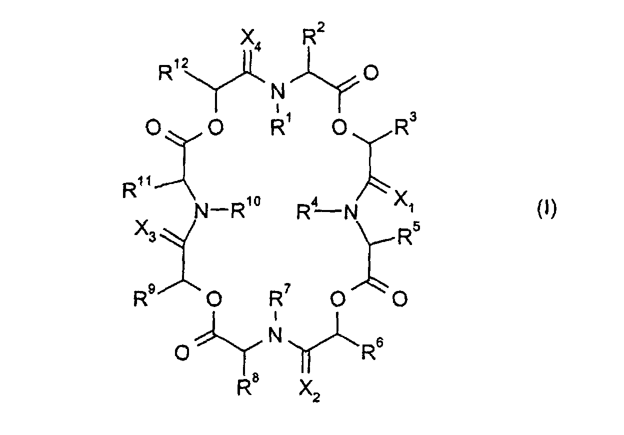 in welcher
R¹, R⁴, R⁷ und R¹⁰ für C₁₋₄-Alkyl, stehen,
R², R⁵, R⁸ und R¹¹ für C₁₋₄-Alkyl, stehen,
R³ und R⁹ für C₁₋₄-Alkyl, stehen,
R⁶ und R¹² unabhängig voneinander für C₁₋₄-Alkyl, Benzothiazol-2-yl-methyl, C₁-C₄- Alkoxycarbonylmethyl, Diphenylmethoxycarbonylmethyl und für gegebenenfalls substituiertes Benzyl, wobei als Substituenten genannt seien Wasserstoff, Halogen, Cyan, Carbamoyl, C₁₋₄-Alkyl, eine Schutzgruppe tragendes Hydroxy oder Hydroxy, C₁₋₈-Alkoxy, Triphenylmethoxy, C₁₋₄-Alkoxy-C₁₋₄-alkoxy, C₂₋₄-Alkenyloxy;
Hetaryl-C₁₋₄-alkoxy, wobei die Heterocyclen wiederum mit Substituenten aus der Reihe C₁₋₄-Alkyl, C₃₋₆-Cycloalkyl, Halogen tragen können, ausgewählt aus: Fur-2-yl-methoxy, Fur-3-yl-methoxy, Tetrahydrofur-2-yl-methoxy, N-Boc-pyrrolidin-2-yl-methoxy, Pyrrolidin-2-yl-methoxy, 5-sec-Butyl-1,2,4-oxadiazol-3-yl-pyridyl-methoxy, 5-Cyclopropyl-1,2,4-oxadiazol-3-ylmethoxy, Imidazol-5-yl-methoxy, Thiazolyl-methoxy, Tetrazol-5-ylmethoxy;
Nitro, eine Schutzgruppe tragendes Amino oder Amino, eine Schutzgruppe tragendes Amino-C₁₋₆-alkoxy oder Amino-C₁₋₆-alkoxy, N-Mono-C₁₋₆alkylamino-C₁₋₆-alkoxy, N,N-Di-C₁₋₆-alkylamino-C₁₋₆-alkoxy, N,N-Di-[(C₁₋₆alkoxy-C₁₋₆-alkyl)]amino-C₁₋₆-alkoxy, C₁₋₄-Alkylamino, C₁₋₄-Dialkylamino, C₃₋₇-Cycloalkylamino- (wobei als ringbildende Atome ein oder mehr Stickstoffatome enthalten und darüber hinaus Sauerstoff- oder Schwefelatome enthalten sein können) C₁₋₆-alkoxy, 2-N-Morpholino-ethoxy, 2-N-Piperidino-ethoxy, Pyrrolidinomethoxy, C₃₋₇-Cyclo-alkylamino- (wobei als ringbildende Atome ein oder mehr Stickstoffatome enthalten und darüber hinaus Sauerstoff- oder Schwefelatome enthalten sein können), C₃₋₇-Cycloalkylamino- (wobei als ringbildende Atome ein oder mehr Stickstoffatome enthalten und darüber hinaus Sauerstoff- oder Schwefelatome enthalten sein können) sulfonyl, N-(N¹-Methyl-piperazino)-sulfonyl, N-(N¹-Methoxycarbonyl-piperazino)-sulfonyl, N¹-Imidazolyl-sulfonyl, N,N-Di-[(C₁₋₆-alkoxy-C₁₋₆-alkyl)]amino-sulfonyl, C₃₋₇-Cyclo-alkylamino- (wobei als ringbildende Atome ein oder mehr Stickstoffatome enthalten und darüber hinaus Sauerstoff- oder Schwefelatome enthalten sein können)-C₁₋₆-alkylamino-sulfonyl, C₁₋₄-Dialkylaminosulfonyl, Sulfamidyl,
und
(i)
X¹ Schwefel bedeutet,
X², X³ und X⁴ unabhängig voneinander Sauerstoff oder Schwefel bedeuten,
oder
(ii)
X² Schwefel bedeutet,
X¹, X³ und X⁴ unabhängig voneinander Sauerstoff oder Schwefel bedeuten,
oder
(iii)
X³ Schwefel bedeutet,
X¹, X² und X⁴ unabhängig voneinander Sauerstoff oder Schwefel bedeuten,
oder
(iv)
X⁴ Schwefel bedeutet,
X¹, X² und X³ unabhängig voneinander Sauerstoff oder Schwefel bedeuten,
sowie deren optische Isomere und Racemate.

2. Verfahren zur Herstellung der Thiodepsipeptide der Formel (I), deren optische Isomeren, Racemate und Salze gemäß Anspruch 1,
**dadurch gekennzeichnet, daß** man die Depsipeptide der allgemeinen Formel (II) und deren Salze, 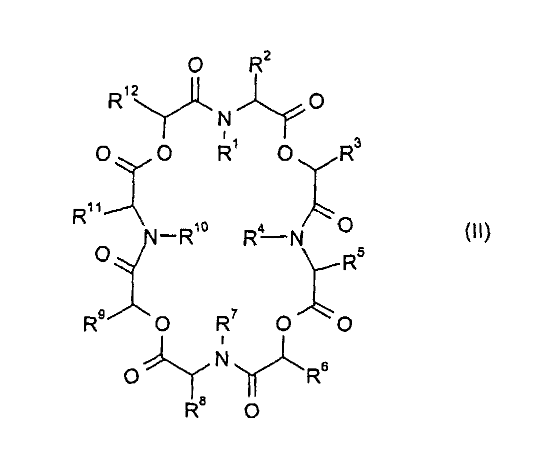 in welcher
R¹ bis R¹² und X¹ bis X⁴ die oben angegebenen Bedeutungen besitzen,
in Gegenwart eines geeigneten Schweflungsagens und in Gegenwart eines geeigneten Verdünnungsmittels thioniert.

3. Cyclische Thiodepsipeptide der Formel (I) gemäß Anspruch 1 zur Bekämpfung von Endoparasiten.

4. Verwendung von cyclischen Thiodepsipeptiden der Formel (I) gemäß Anspruch 1 zur Herstellung von endoparasitiziden Mitteln.

5. Endoparasitizide Mittel **gekennzeichnet durch** einen Gehalt an Thiodepsipeptiden der Formel (I) gemäß Anspruch 1 gegebenenfalls in Mischung mit üblichen Verdünnungs- und Zusatzstoffen.

## Claims

1. Process for preparing the thiodepsipeptides of the formula (I) and their optical isomers, racemates and salts according to Claim 1,
**characterized in that** the depsipeptides of the general formula (II) and salts thereof 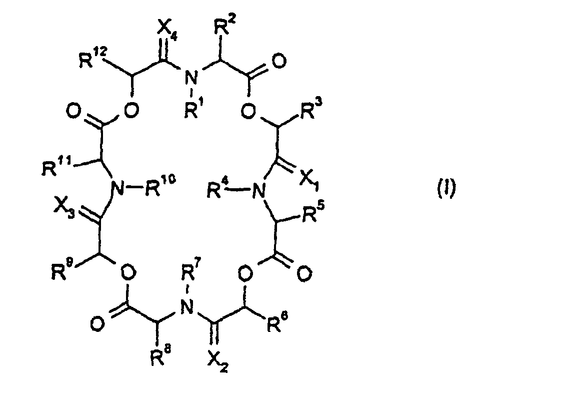 in which
R¹ to R¹² and X¹ to X⁴ are each as defined above,
are thionated in the presence of a suitable sulfurizing agent and in the presence of a suitable diluent.

2. Compounds of the general formula (I) and salts thereof 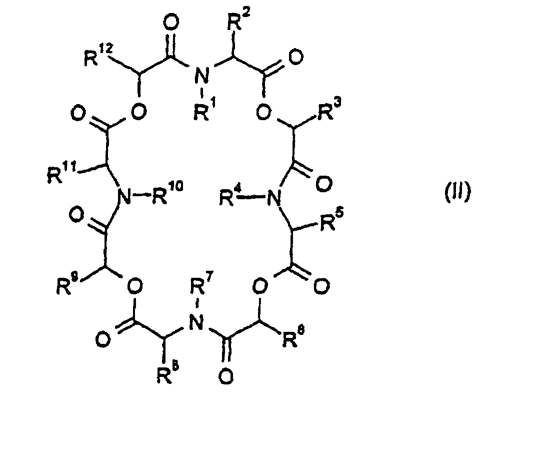 in which
R¹, R⁴, R⁷ and R¹⁰ each represent C₁₋₄-alkyl,
R², R⁵, R⁸ and R¹¹ each represent C₁₋₄-alkyl, R³ and R⁹ each represent C₁₋₄-alkyl,
R⁶ and R¹² each represent independently of one another C₁₋₄-alkyl, benzothiazol-2-yl-methyl, C₁₋C₄-alkoxycarbonylmethyl, diphenylmethoxycarbonylmethyl, and optionally substituted benzyl, possible substituents including hydrogen, halogen, cyano, carbamoyl, C₁₋₄-alkyl, hydroxyl or hydroxyl carrying a protecting group, C₁₋₈-alkoxy, triphenylmethoxy, C₁₋₄-alkoxy-C₁₋₄-alkoxy, C₂₋₄-alkenyloxy;
hetaryl-C₁₋₄-alkoxy where the heterocycles again may carry with substituents from the group consisting of C₁₋₄-alkyl, C₃₋₆-cycloalkyl, halogen, selected from the group comprising fur-2-yl-methoxy, four-3-yl-methoxy, tetrahydrofur-2-yl-methoxy, N-boc-pyrrolidin-2-yl-methoxy, pyrrolidin-2-yl-methoxy, 5-sec-butyl-1,2,4-oxadiazol-3-yl-pyridyl-methoxy, 5-cyclopropyl-1,2,4-oxadiazol-3-yl-methoxy, imidazol-5-yl-methoxy, thiazolyl-methoxy and tetrazol-5-yl-methoxy;
nitro, amino or amino carrying a protecting group, amino-C₁₋₆-alkoxy or amino-C₁₋₆-alkoxy carrying a protecting group, N-mono-C₁₋₆-alkylamino-C₁₋₆-alkoxy, N,N-di-C₁₋₆-alkylamino-C₁₋₆-alkoxy, N,N-Di-[(C₁₋₆-alkoxy-C₁₋₆-alkyl)]amino-C₁₋₆-alkoxy, C₁₋₄-alkylamino, C₁₋₄-dialkylamino, C₃₋₇-cycloalkylamino- (which may contain one or more nitrogen atoms as ring-forming atoms and additionally oxygen or sulfur atoms) C₁₋₆-alkoxy, 2-N-morpholino-ethoxy, 2-N-piperidino-ethoxy, pyrrolidinomethoxy, C₃₋₇-cyclo-alkylamino- (which may contain one or more nitrogen atoms as ring-forming atoms and additionally oxygen or sulfur atoms), C₃₋₇-cyclo-alkylamino-(which may contain one or more nitrogen atoms as ring-forming atoms and additionally oxygen or sulfur atoms) sulfonyl, N-(N¹-methyl-piperazino)-sulfonyl, N-(N¹-methoxy-carbonyl-piperazino)-sulfonyl, N¹-imidazolyl-sulfonyl, N,N-di[(C₁₋₆-alkoxy-C₁₋₆-alkyl)]amino-sulfonyl, C₃₋₇-cyclo-alkylamino- (which may contain one or more nitrogen atoms as ring-forming atoms and additionally oxygen or sulfur atoms)-C₁₋₆-alkylaminosulfonyl, C₁₋₄-dialkylamino-sulfonyl, sulfamidyl,
and
(i)
X¹ represents sulfur,
X², X³ and X⁴ each represent independently of one another oxygen or sulfur,
or
(ii)
X² represents sulfur,
X¹, X³ and X⁴ each represent independently of one another oxygen or sulfur,
or
(iii)
X³ represents sulfur,
X¹, X² and X⁴ each represent independently of one another oxygen or X¹, X² and X⁴ each represent independently of one another oxygen or sulfur,
or
(iv)
X⁴ represents sulfur,
X¹, X² and X³ each represent independently of one another oxygen or sulfur,
and their optical isomers and racemates.

3. Cyclic thiodepsipeptides of the formula (I) according to Claim 1 for controlling endoparasites.

4. Use of cyclic thiodepsipeptides of the formula (I) according to Claim 1 for preparing endoparasiticidal compositions.

5. Endoparasiticidal compositions, **characterized by** a content of thiodepsipeptides of the formula (I) according to Claim 1, if appropriate as a mixture with conventional diluents and additives.

## Revendications

1. Composés de formule générale (I) et leurs sels : 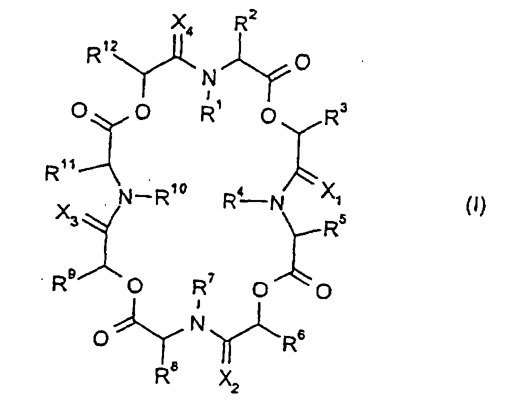 formule dans laquelle
R¹, R⁴, R⁷ et R¹⁰ représentent un reste alkyle en C₁-C₄,
R², R⁵, R⁸ et R¹¹ représentent un reste alkyle en C₁-C₄,
R³ et R⁹ représentent un reste alkyle en C₁-C₄,
R⁶ et R¹² représentent, indépendamment l'un de l'autre, un reste alkyle en C₁-C₄, benzothiazole-2-yl-méthyle, (alkoxy en C₁-C₄)carbonylméthyle, diphénylméthoxycarbonylméthyle et un reste benzyle éventuellement substitué, les substituants que l'on peut mentionner étant l'hydrogène, un halogène, un radical cyano, carbamoyle, alkyle en C₁-C₄, un radical hydroxy portant un groupe protecteur ou un radical hydroxy, alkoxy en C₁-C₈, triphénylméthoxy, (alkoxy en C₁-C₄) - (alkoxy en C₁-C₄), alcényloxy en C₂-C₄ ;
un reste hétaryl-(alkoxy en C₁-C₄), dont les hétérocycles peuvent porter là encore des substituants de la série alkyle en C₁-C₄, cycloalkyle en C₃-C₆ ou halogéno, choisis entre les restes : fur-2-ylméthoxy, fur-3-ylméthoxy, tétrahydrofur-2-ylméthoxy, N-Boc-pyrrolidine-2-ylméthoxy, pyrrolidine-2-ylméthoxy, 5-sec.-butyl-1,2,4-oxadiazole-3-ylpyridylméthoxy, 5-cyclopropyl-1,2,4-oxadiazole-3-ylméthoxy, imidazole-5-ylméthoxy, thiazolylméthoxy, tétrazole-5-ylméthoxy ;
un reste nitro, amino porteur ou non d'un groupe protecteur, amino-(alkoxy en C₁-C₆) porteur ou non d'un groupe protecteur, N-mono-(alkyle en C₁-C₆)amino-(alkoxy en C₁-C₆), N,N-di-(alkyle en C₁-C₆)-amino(alkoxy en C₁-C₆), N,N-di-[(alkoxy en C₁-C₆) - (alkyle en C₁-C₆)]amino(alkoxy en C₁-C₆), alkylamino en C₁-C₄, di-(alkyle en C₁-C₄)amino, (cycloalkyle en C₃-C₇)amino-(alkoxy en C₁-C₆) (un ou plusieurs atomes d'azote et en outre des atomes d'oxygène ou de soufre pouvant être présents comme atomes formant le noyau), 2-N-morpholinoéthoxy, 2-N-pipéridinoéthoxy, pyrrolidinométhoxy, cycloalkylamino en C₃ à C₇ (un ou plusieurs atomes d'azote et en outre des atomes d'oxygène ou de soufre pouvant être présents comme atomes formant le noyau), (cycloalkyle en C₃-C₇) amino-sulfonyle (un ou plusieurs atomes d'azote et en outre des atomes d'oxygène ou de soufre pouvant être présents comme atomes formant le noyau), N-(N¹-méthylpipérazino)sulfonyle, N-(N¹-méthoxycarbonylpipérazino)sulfonyle, N¹-imidazolylsulfonyle, N,N-di-[(alkoxy en C₁-C₆) - (alkyle en C₁-C₆)]aminosulfonyle, (cycloalkyle en C₃-C₇)amino-alkylaminosulfonyle en C₁-C₆ (un ou plusieurs atomes d'azote et en outre des atomes d'oxygène ou de soufre pouvant être présents comme atomes formant le noyau), di(alkyle en C₁-C₄)aminosulfonyle, sulfamidyle,
et
(i)
X¹ est le soufre,
X², X³ et X⁴ représentent indépendamment l'un de l'autre l'oxygène ou le soufre,
ou bien
(ii)
X² est le soufre
X¹, X³ et X⁴ représentent indépendamment l'un de l'autre l'oxygène ou le soufre,
ou bien
(iii)
X³ est le soufre,
X¹, X² et X⁴ représentent indépendamment l'un de l'autre l'oxygène ou le soufre,
ou bien
(iv)
X⁴ est le soufre,
X¹, X² et X³ représentent indépendamment l'un de l'autre l'oxygène ou le soufre,
ainsi que leurs isomères optiques et leurs racémates.

2. Procédé de production des thiodepsipeptides de formule (I), de leurs isomères optiques, de leurs racémates et de leurs sels suivant la revendication 1, **caractérisé en ce qu'**on conduit la sulfuration des depsipeptides de formule générale (II) et de leurs sels, 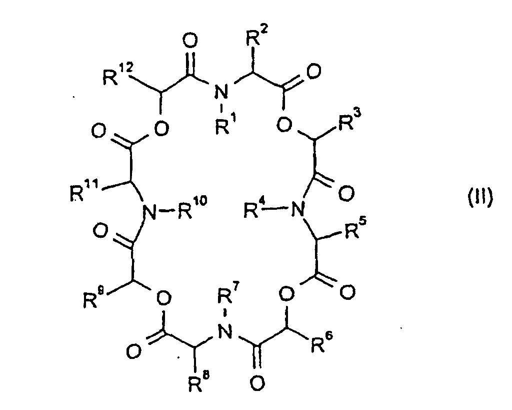 formule dans laquelle
R¹ à R¹² et X¹ à X⁴ ont les définitions indiquées ci-dessus,
en présence d'un agent de sulfuration approprié et en présence d'un diluant approprié.

3. Thiodepsipeptides cycliques de formule (I) suivant la revendication 1, destinés à combattre des endoparasites.

4. Utilisation de thiodepsipeptides cycliques de formule (I) suivant la revendication 1 pour la préparation de compositions endoparasiticides.

5. Compositions endoparasiticides, **caractérisées par** une teneur en thiodepsipeptides de formule (I) suivant la revendication 1, le cas échéant en mélange avec des diluants et des additifs classiques.
